# EUROPEAN PATENT APPLICATION

(11) **EP 1 955 715 A1**
(43) Date of publication of application: **13.08.2008**
(21) Application number: 07102070.5
(22) Date of filing: 09.02.2007
(51) Int. Cl.: A61K 48/00, A61K 38/46, A61P 43/00, G01N 33/573, C12Q 1/34

(54) **Use of Sirt7 for treating age-related diseases**

(71) Applicant: Max-Planck-Gesellschaft zur Förderung der Wissenschaften e.V., 80539 München (DE)
(72) Inventor: Braun, Thomas, Bad Nauheim 61231 (DE); Bober, Eva, Bad Nauheim 61231 (DE); Vakhrushva, Olesya, Bad Nauheim 61231 (DE)
(74) Representative: Vossius & Partner

(57) **Abstract**

The present invention relates to a pharmaceutical composition comprising (i) a nucleic acid molecule encoding a protein having Sirt7 function wherein said nucleic acid molecule (a) has the sequence of SEQ ID NO: 1, 3, 5 or 7; (b) encodes a protein having the sequence of SEQ ID NO: 2, 4, 6 or 8; (c) hybridizes under stringent conditions to the molecule of (a) or (b); or (d) has an identity on the nucleic acid level of at least 80% with the molecule of (a), (b) or (c); (ii) a vector comprising the nucleic acid molecule of (i); (iii) a host cell comprising the vector of (ii); or (iv) a protein encoded by the nucleic acid molecule of (i). The pharmaceutical composition is used for example in the treatment of age-related diseases. Furthermore, the invention relates to screening methods for the identification of compounds useful in the treatment of age-related diseases.

## Description

The present invention relates to a pharmaceutical composition comprising (i) a nucleic acid molecule encoding a protein having Sirt7 function wherein said nucleic acid molecule (a) has the sequence of SEQ ID NO: 1, 3, 5 or 7; (b) encodes a protein having the sequence of SEQ ID NO: 2, 4, 6 or 8; (c) hybridizes under stringent conditions to the molecule of (a) or (b); or (d) has an identity on the nucleic acid level of at least 80% with the molecule of (a), (b) or (c); (ii) a vector comprising the nucleic acid molecule of (i); (iii) a host cell comprising the vector of (ii); or (iv) a protein encoded by the nucleic acid molecule of (i). The pharmaceutical composition may be used, for example, in the treatment of age-related diseases. Furthermore, the invention relates to screening methods for the identification of compounds useful in the treatment of age-related diseases.

Throughout this specification, several documents are cited. The disclosure content of these documents is herewith incorporated by reference (including all product descriptions and manufacturers instructions).

As the progress in modern medicine has lead to a tremendous increase in lifespan in the industrialized countries, the need for the treatment or amelioration of age-related diseases has concurrently increased since a growing part of the population suffers from such diseases. Several genes that are involved in aging and lifespan control have been proposed. These so-called longevity genes are often involved in pathways that guarantee better survival under conditions such as food deprivation, cold or other types of environmental stress (Porcu and Chiarugi Trends in Pharmacological Sciences 2005, 26: 94).

One example of such longevity genes are the silent information regulator (SIR) genes of the budding yeast S. cerevisiae. These are nonessential genes required for transcriptional repression of several genomic loci. Among these genes, Sir2 is unique since it belongs to a large family of closely related proteins present in eukaryotic as well as in prokaryotic species. Sir2 is responsible for promoting longevity in yeast mother cells by inhibiting recombination in the rDNA (ribosomal DNA) repeats since the recombinational excision and subsequent accumulation of extrachromosomal rDNA circles is involved in senescence. In addition, Sir2 family members have been suggested to mediate the lifespan extending effects of caloric restriction, a mechanism which is capable of extending the lifespan of a diverse range of organisms ranging from yeast to mammals (Mostoslavsky et al. Cell 2006, 124: 315).

The fact that several mechanisms that regulate lifespan are well conserved across species (e.g. caloric restriction) led to the speculation that mammalian Sir2 family members are also involved in lifespan regulation in mammals (North and Verdin Genome Biology 2004, 5: 224).

There are seven mammalian Sir2 family members which are named Sirt1 to Sirt7 wherein Sirt1 exhibits the closest relationship to Sir2. In contrast to Sir2 which exclusively deacetylates histones, Sirt1 has a diverse list of substrates. Although the lifetime prolonging effects of Sirt1 have not unambiguously been shown in mammals, its role in the improvement of stress resistance and in the modulation of the metabolism in other model organisms led to the assumption that it plays an important role in the regulation of lifespan even in mammals. Two of the Sirt1 target genes, namely p53 and PGC-1α mediate longevity associated effects: p53 is deacetylated by Sirt1 leading to a decreased rate of stress-induced apoptosis and PGC-1α forms a complex with Sirt1 thereby promoting gluconeogenesis in liver and free fatty acid mobilization upon fasting. This effect leads to the inhibition of insulin/IGF signalling and seems to be conserved between yeast and mammals. Inhibition of this signalling pathway appears to be also responsible for the lifespan extending effects of caloric restriction (Mostoslavsky et al. Cell 2006, 124: 315).

Only little is known about the longevity related functions of other mammalian Sirts although recent data indicate the involvement of Sirt6 in genomic stabilization (Mostoslavsky et al. Cell 2006, 124: 315). Almost nothing is known with respect to Sirt7. As pointed out in Porcu and Chiarugi (2005), Sirt7 does not show any deacetylase activity. Hence, the prior art clearly teaches away from the involvement of Sirt7 in processes that regulate ageing based on a reduced p53 activity. Furthermore, it has also been postulated that Sirt7 cannot exhibit a deacetylase activity because it contains a serine residue at position 115 instead of a glycine in the enzymatic core domain whereas the prior art describes that the glycine residue is essential for the deacetylating activity (Imai et al. Nature 2002, 403: 795; Michita et al. Mol. Biol. Cell 2005, 16: 4623; North et al. Mol. Cell 2003, 11: 437).

Accordingly, the prior art clearly teaches away from the use of Sirt7 as a target for pharmaceutical interventions for treating age-related diseases. In view of the ageing community the technical problem underlying the present invention was the provision of means and methods for treating age-related diseases.

The solution to this problem is achieved by providing the embodiments as characterized in the claims.

Accordingly, the present invention relates to a pharmaceutical composition comprising (i) a nucleic acid molecule encoding a protein having Sirt7 function wherein said nucleic acid molecule (a) has the sequence of SEQ ID NO: 1, 3, 5 or 7; (b) encodes a protein having the sequence of SEQ ID NO: 2,4,6 or 8; (c) hybridizes under stringent conditions to the molecule of (a) or (b); or (d) has an identity on the nucleic acid level of at least 80% with the molecule of (a), (b) or (c); (ii) a vector comprising the nucleic acid molecule of (i); (iii) a host cell comprising the vector of (ii); or (iv) a protein encoded by the nucleic acid molecule of (i).

In accordance with the present invention, the term "pharmaceutical composition" relates to a composition for administration to a patient, preferably a human patient. The pharmaceutical composition of the invention comprises the compounds recited above. It may, optionally, comprise further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). The pharmaceutical composition of the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known conventional methods. These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g per day. However, a more preferred dosage might be in the range of 0.01 mg to 100 mg, even more preferably 0.01 mg to 50 mg and most preferably 0.01 mg to 10 mg per day.

"Nucleic acid molecules", in accordance with the present invention, include DNA, such as cDNA or genomic DNA, and RNA. Further included are nucleic acid mimicking molecules known in the art such as synthetic or semi-synthetic derivatives of DNA or RNA and mixed polymers, both sense and anti-sense strands. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. They may contain additional non-natural or derivative nucleotide bases, as will be readily appreciated by those skilled in the art.

In a preferred embodiment the nucleic acid molecule(s) is/are DNA.

"A protein having Sirt7 function" in accordance with the present invention is a protein exhibiting for example deacetylation of p53. Furthermore, said protein may also be capable of deacetylating FOXO, NFκB or p300, downregulating PEPCK, activating FAS and de-phosphorylating p38.

As regards the nucleic acid molecule referred to in (i) above, the sequence of SEQ ID NO: 1 and 3 relates to the Sirt7 nucleic acid sequence of mouse, the sequence of SEQ ID NO: 5 relates to the Sirt7 nucleic acid sequence of rat and the sequence of SEQ ID NO: 7 relates to the Sirt7 nucleic acid sequence of human.

The nucleic acid molecule referred to in (i) above encodes for example a protein having the sequence of SEQ ID NO: 2 and 4 which relates to the Sirt7 amino acid sequence of mouse, or having the sequence of SEQ ID NO: 6 which relates to the Sirt7 amino acid sequence of rat or having the sequence of SEQ ID NO: 8 which relates to the Sirt7 amino acid sequence of human.

The nucleic acid molecule used in the pharmaceutical composition may be at least 80% identical, preferably at least 90% and more preferably at least 95% identical to the nucleic acid molecule of (i)(a) through (i)(c). It is of note that in mice (North and Verdin, Genome Biology 2004, 5: 224) and rats the amino acids N at position 169 and H at position 188 in SEQ ID NO: 2 are required for the deacetylating activity of the protein while in humans the amino acids N168 and H187 are required. Thus, the nucleotide positions corresponding to these amino acids should preferably be retained when using the aforementioned nucleic acid molecules with the named identity values. Such molecules may be homologous molecules from other species, such as homologs or mutated sequences to mention the most prominent examples. To evaluate the identity level between two nucleotide or protein sequences, they can be aligned electronically using suitable computer programs known in the art. Such programs comprise BLAST (Altschul et al., J. Mol. Biol. 1990, 215: 403), variants thereof such as WU-BLAST (Altschul & Gish, Methods Enzymol. 1996, 266: 460), FASTA (Pearson & Lipman, Proc. Natl. Acad. Sci. USA 1988, 85: 2444) or implementations of the Smith-Waterman algorithm (SSEARCH, Smith & Waterman, J. Mol. Biol. 1981, 147: 195). These programs, in addition to providing a pairwise sequence alignment, also report the sequence identity level (usually in percent identity) and the probability for the occurrence of the alignment by chance (P-value).

Programs such as CLUSTALW (Higgins et al., Nucleic Acids Res. 1994, 22: 4673) can be used to align more than two sequences.

The term "hybridizes/hybridizing" as used herein refers to a pairing of a polynucleotide to a (partially) complementary strand of this polynucleotide which thereby form a hybrid.

It is well known in the art how to perform hybridization experiments with nucleic acid molecules. Correspondingly, the person skilled in the art knows what hybridization conditions she/he has to use to allow for a successful hybridization in accordance with item (i)(c), above. The establishment of suitable hybridization conditions is referred to in standard text books such as Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley lnterscience, N.Y. (1989), or Higgins and Hames (Eds.) "Nucleic acid hybridization, a practical approach" IRL Press Oxford, Washington DC, (1985).

"Stringent conditions" refers to hybridization conditions which comprise, e.g. on overnight incubation at 65°C in 4x SSC (600 mM NaCl, 60 mM sodium citrate) followed by washing at 65°C in 0.1x SSC for one hour. Alternatively, hybridization conditions can comprise: an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing in e.g. 0.1-0.5x SSC at about 55-65°C for about 5 to 20 min. Said conditions for hybridization are also known by a person skilled in the art as "highly stringent conditions for hybridization". Also contemplated are nucleic acid molecules that hybridize to the polynucleotides of the invention at lower stringency hybridization conditions ("low stringency conditions for hybridization"). Changes in the stringency of hybridization are primarily accomplished through the manipulation of formamide concentration (lower percentages of formamide result in lowered stringency), salt conditions, or temperature. For example, lower stringency conditions include an overnight incubation at 50°C in 4x SSC or an overnight incubation at 37°C in a solution comprising 6x SSPE (20x SSPE = 3M NaCl; 0.2M NaH2PO4; 0.02M EDTA, pH 7.4), 0.5% SDS, 30% formamide, 100 mg/ml salmon sperm blocking DNA; followed by washes at 50°C with 1 x SSPE, 0.1% SDS. In addition, to achieve an even lower stringency, washes performed following stringent hybridization can be done at higher salt concentrations (e.g. 5x SSC). It is of note that variations in the above conditions may be accomplished through the inclusion and/or substitution of alternate blocking reagents. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado. The embodiment recited herein above preferably refers to highly stringent conditions and alternatively to conditions of lower stringency.

The biochemical functions of the mammalian sirtuins have predominantly been studied for Sirt1 which is the closest homologue of the yeast Sir2 which is known to increase the lifespan of yeast by about 30% (Kaeberlein et al. Genes & Dev. 1999, 13: 2570) and of C. elegans by about 50% (Tissenbaum & Guarente Nature 2001, 410: 227) when present in more than one copy. Several potential target proteins for a Sirt1-dependent deacetylation have been postulated among which are p53, FOXO, NFkB and p300. However, no biologically relevant deacetylation could be demonstrated in vivo. In addition, no unambiguous age-preventive function of Sirt1 has been documented in vivo either. Knock-out of the Sirt1 gene leads to early postnatal lethality. The only sirtuin which appears to be also involved in age-related processes may be Sirt6. However, Sirt6-deficient mice die during the first postnatal month. It turned out that the mechanism underlying the Sirt6 function is the maintenance of genomic stability (Mostoslavsky et al. Cell 2006, 124: 315). In the context of the present invention, it has been surprisingly discovered that Sirt7 is the first mammalian sirtuin which is involved in ageing processes. It plays a major role in the regulation of such processes by improving the ability to respond to different stressors. Of great importance is the discovery that two pathophysiological aberrations, which occur with progressing age in humans, also occur in Sirt7-deficient mice, namely general chronic inflammation and inflammatory cardiomyopathy (see Examples 1 and 4). It was further found that Sirt7 regulates p53 and various genes that control metabolic pathways (see Examples 2 and 5). Deletion of the Sirt7 gene in a mouse in vivo model led to premature aging, reduced ability to respond to adverse living conditions and cardiac disease (Examples 3 to 5). Resveratrol, a substance present in red wine grapes and believed to be responsible for an increased lifespan in red-wine consumers, has been reported to specifically induce Sirt1 (Baur & Sinclair Nature 2006, Rev. 5: 493) However, the present invention demonstrates that resveratrol is an even more potent inducer of Sirt7 (see Example 2). In accordance with this finding, it is postulated that it is the effect of resveratrol on Sirt7 and not on Sirt1 which counts for the lifespan extending characteristics of this compound. Furthermore, it has been found in the context of the present invention that Sirt7-/- mice exhibit an increase in the Akt/PKB and ras/raf signalling pathways which is accompanied by heart hypertrophy (see Example 5). It is therefore postulated that Sirt7 may inhibit the insulin/IGF pathway which is believed to be the most important lifespan extending mechanism in lower organisms. Accordingly, Sirt7 was identified as a target to prevent age-related diseases either through stimulation of its expression or its enzymatic activities. Furthermore, Sirt7 may even be directly used in pharmaceutical compositions as recited hereinabove to treat age-related diseases. Sirt7 is a unique target for such interventions since other members of the Sirt gene family are involved in developmental processes like Sirt1 or in the maintenance of genomic stability like Sirt6. Consequently, only the use of Sirt7 is promising with respect to the treatment of age-related diseases since it does not interfere with other fundamental, biological processes.

The present invention also relates to a pharmaceutical composition comprising a vector comprising the nucleic acid molecule referred to above. Preferably, the vector is a plasmid, cosmid, virus, bacteriophage or another vector conventionally used e.g. in genetic engineering. Incorporation of the nucleic acid into a vector offers the possibility of introducing the nucleic acid molecule efficiently into the cells and preferably the DNA of a recipient. The recipient may be a single cell such as cell from a cell line. Such a measure renders it possible to express, when expression vectors are chosen, the respective nucleic acid molecule in the recipient. Thus, incorporation of the nucleic acid molecule into an expression vector opens up the way to a permanently elevated level of the encoded protein in any cell or a subset of selected cells of the recipient.

In a preferred embodiment, the recipient is a mammal. In a more preferred embodiment, the mammal is a human.

The nucleic acid molecule may be inserted into several commercially available vectors. Non-limiting examples include vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen), pCINeo (Promega), Okayama-Berg cDNA expression vector pcDV1 (Pharmacia), pRc/CMV, pcDNA1, pSPORT1 (GIBCO BRL), pGEMHE (Promega), pSVL and pMSG (Pharmacia, Uppsala, Sweden), pRSVcat (ATCC 37152), pSV2dhfr (ATCC 37146) or pBC12MI (ATCC 67109).

The nucleic acid molecule referred to above may also be inserted into vectors such that a translational fusion with another nucleic acid molecule is generated. The vectors may also contain an additional expressible polynucleotide coding for one or more chaperones to facilitate correct protein folding.

For vector modification techniques, see Sambrook and Russel (2001), loc. cit. Generally, vectors can contain one or more origin of replication (ori) and inheritance systems for cloning or expression, one or more markers for selection in the host, e. g., antibiotic resistance, and one or more expression cassettes.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in eukaryotic cells are well known to those skilled in the art. These elements comprise regulatory sequences ensuring the initiation of the transcription (e. g. translation initiation codon, promoters, enhancers, and/or insulators), internal ribosomal entry sites (IRES) (Owens Proc. Natl. Acad. Sci. USA 2001, 98: 1471) and optionally poly-A signals ensuring termination of transcription and stabilization of the transcript. Additional regulatory elements may include transcriptional as well as translational enhancers, and/or naturally-associated or heterologous promoter regions. Preferably, the nucleic acid molecule is operatively linked to such expression control sequences allowing expression in eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Possible examples for regulatory elements ensuring the initiation of transcription comprise the cytomegalovirus (CMV) promoter, SV40-promoter, RSV-promoter (Rous sarcome virus), the lacZ promoter, the gai10 promoter, human elongation factor 1a-promoter, CMV enhancer, CaM-kinase promoter, the Autographa californica multiple nuclear polyhedrosis virus (AcMNPV) polyhedral promoter or the SV40-enhancer. Examples for further regulatory elements in prokaryotes and eukaryotic cells comprise transcription termination signals, such as SV40-poly-A site or the tk-poly-A site or the SV40, lacZ and AcMNPV polyhedral polyadenylation signals, downstream of the polynucleotide. Moreover, elements such as origin of replication, drug resistance gene, regulators (as part of an inducible promoter) may also be included. Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter).

The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al., Biochem J. 1991, 227:277; Bebbington et al., Bio/Technology 1992, 10:109). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture.

The nucleic acid molecules as described hereinabove may be designed for direct introduction or for introduction via liposomes, phage vectors or viral vectors (e.g. adenoviral, retroviral) into the cell. Additionally, baculoviral systems or systems based on Vaccinia Virus or Semliki Forest Virus can be used as eukaryotic expression system for the nucleic acid molecules of the invention.

Mammalian host cells that could be used include, human Hela, HEK293, H9 and Jurkat cells, mouse NIH3T3 and C127 cells, Cos 1, Cos 7 and CV1, quail QC1-3 cells, mouse L cells and Chinese hamster ovary (CHO) cells. Also within the scope of the present invention are primary mammalian cells such as mouse embryonic fibroblasts (MEF). Alternatively, the recombinant (poly)peptide can be expressed in stable cell lines that contain the gene construct integrated into a chromosome.

In another embodiment, the present invention relates to the use of (i) a nucleic acid molecule encoding a protein having Sirt7 function, wherein said nucleic acid molecule (a) has the sequence of SEQ ID NO: 1, 3, 5 or 7; (b) encodes a protein having the sequence of SEQ ID NO: 2, 4, 6 or 8; (c) hybridizes under stringent conditions to the molecule of (a) or (b); or (d) has an identity on the nucleic acid level of at least 80% with the molecule of (a), (b) or (c); (ii) a vector comprising the nucleic acid molecule of (i); (iii) a host cell comprising the vector of (ii); or (iv) a protein encoded by the nucleic acid molecule of (i) for the manufacture of a pharmaceutical composition for treating age-related diseases.

As described hereinabove, Sirt7 protein, or its corresponding nucleic acid molecule as described above, is a key regulator of age-related processes. Therefore, its use in the preparation of pharmaceutical compositions for the treatment of age-related diseases offers the possibility to specifically target diseases which accompany ageing processes.

The term "age-related diseases" as used herein refers to diseases which can be characterized by the loss of a cell's, organ's or organism's peak function that continues until its failure and death. In humans most physiological functions such as hearing, eyesight, taste, lung capacity, agility, immune response, adaptation to change etc. reach peak ability between the ages of 11 and 20 years old. After that age there is a slow decline in performance as the degenerative process of aging begins. Age-related diseases are well known in the literature, for example in Cutler & Mattson (Ageing Res. Rev. 2006, 5: 221).

In a preferred embodiment, the age-related disease is selected from the group consisting of fibrosis, inflammatory cardiomyopathy, heart hypertrophy, liver degeneration, skeletal muscle degeneration and chronic general inflammation.

Fibrosis generally relates to the formation of scar tissue in organs such as the liver or the heart. Inflammatory cardiomyopathy relates to an abnormal heart condition in which the heart is dilated (poor pumping power), restrictive (impaired ability of the heart to fill) or hypertrophic (enlarged heart). Heart hypertrophy is a disorder in which the heart muscle is so strong that it does not relax enough to fill the heart with blood and so has reduced pumping ability. Skeletal muscle degeneration generally refers to muscle wasting, sarcopenia as well as depletion of muscular tissues and fat stores. Chronic general inflammation generally relates to an infiltration of organs with inflammatory cells such as macrophages and neutrophile lymphocytes as well as increased levels of CRP and IL6. Emerging pathological evidence indicates that major chronic ageing-related diseases such as atherosclerosis, arthritis, dementia, osteoporosis and cardiovascular diseases are inflammation-related.

In another embodiment, the present invention relates to a method for the identification of a compound useful in the treatment of age-related diseases or as a lead compound for the development of an agent for treating age-related diseases comprising the steps: (a) contacting Sirt7 protein with a test compound and an acetylated Sirt7 substrate; and (b) determining the level of the deacetylated Sirt7 substrate and/or the level of the acetylated Sirt7 substrate before contacting the protein with the test compound and after contacting the protein with the test compound wherein a reduced level of acetylated Sirt7 substrate or an increased level of deacetylated Sirt7 substrate after contacting the protein with the test compound as compared to the level before contacting the protein with the test compound indicates that the test compound is a compound useful in the treatment of age-related diseases or as a lead compound for the development of an agent for treating age-relafied diseases.

Since Sirt7 is, as described hereinabove, involved in processes that regulate ageing, its use as a target for the discovery of compounds that interfere with the processes of ageing is also envisaged by the present invention. Preferably, these compounds activate translation and/or enzymatic activity of Sirt7 in order to increase the level of physiologically active protein. Sirt7 protein is useful, as has been surprisingly found in accordance with the present invention, for the prevention of several diseases which are related to ageing, as is evident from the phenotypic alterations observed in Sirt7 - /- mice (see Example 1 in connection with Examples 3 and 4) and therefore, increase of its expression level by the use of compounds identified in the above-described screen will result in amelioration or even loss of symptoms associated with said diseases.

In another aspect, the increase of enzymatic activity of Sirt7 by compounds identified as described above is also of use in the treatment of age-related diseases since an increased enzymatic activity leads to an increased substrate turnover rate which consequently leads to a reduction of active effector molecules such as p53. This is demonstrated in the reduction of active p53 by deacetylation via Sirt7 in vitro (Example 2). It is known that p53 is involved in premature ageing phenotypes (Gentry and Venkatachalam, Aging Cell, 2005: 4, 157). In Sirt7 -/- mice, p53 was found to be in its active (acetylated) state. Therefore, it is evident that a higher turnover rate of p53 from its acetylated into its deacetylated (inactive) state will lead to reduction of the negative effects caused by active p53. As the applicant does not wish to be bound by any theory, also other mechanisms of action of Sirt7 via other (known or not yet known) effector molecules are within the scope of the present invention.

A "compound" in accordance with the present invention is, for example, a small molecule. Such a small molecule may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively the compound may be an inorganic compound. Inorganic compounds are derived from mineral sources and include all compounds without carbon atoms (except carbon dioxide, carbon monoxide and carbonates).

The "acetylated Sirt7 substrate" in accordance with the present invention includes but is not limited to p53, FOXO, NFkB, p300, MyoD or the neuronal bHLH proteins NSCL1 and NSCL2. Also included are, for example, histone H3 and H4, Ku-70, PVAF, PGC-1α und PPAR_{γ}.

The determination of the level of acetylated or deacetylated Sirt7 substrate is accomplished by methods including but not limited to Western blot analysis, reverse-phase HPLC, Charcoal-binding assays or thin-layer-chromatography assays. For Western Blot analysis antibodies can be used that recognize the acetylated form of the substrate or, alternatively, the substrate may be immunoprecipitated and the acetylation level detected using an antibody against acetylated lysine. Using radiolabeled acetylated substrates, SDS/PAGE gels can also be dried and exposed to photographic film, after which the loss of radioactivity on the substrate can be visualized. Alternatively, the reaction mixture can be spotted onto a Whatman P81 cation exchange paper, which traps the radiolabeled, acetylated protein substrate. After extensive washing, the paper is subjected to scintillation counting, and a decrease in the counts reflects the level of deacetylation. Analysis by reverse-phase HPLC relies on the separation of substrates and products of the deacetylase reaction. Quenched reaction mixtures are injected onto a C18 column and, using a gradient of increased levels of organic solvent, substrates, products, and enzyme can be resolved. Charcoal-binding assays can be performed using 3H acetylated substrate. This assay takes advantage of the fact that under high pH and heat, the 3H acetyl group from 3H-O-acetyl-ADP-ribose (OAADPr) is hydrolysed. The 3H acetate does not bind to charcoal and can, therefore, be separated from the rest of the charcoal-bound substrates and products. Thin-layer-chromatography assays can be performed in presence of radiolabeled NAD+.

Substrate measurement is advantageous since it directly delivers a readout of the enzymatic activity of the investigated protein.

In a preferred embodiment, the Sirt7 substrate is p53.

P53 deacetylation is suitable as a readout for assessing the age-suppressing properties of various compounds since, as surprisingly discovered in the present invention, Sirt7 efficiently deacetylates p53.

In a more preferred embodiment, the method is carried out in vitro.

In vitro methods offer the possibility of establishing high-throughput assays which are capable of screening up to several thousand compounds in parallel. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be deconvoluted to identify the one or more test compounds in said mixture giving rise to said activity.

The identified so-called lead compounds may be optimized to arrive at a compound which may be, for example, used in a pharmaceutical composition. Methods for the optimization of the pharmacological properties of compounds identified in screens, the lead compounds, are known in the art and comprise methods of modifying a compound identified as a lead compound to achieve: (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmacokinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carboxylic acids, or (iii) esterification of hydroxyl groups to, e.g. phosphates, pyrophosphates or sulfates or hemi-succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiff's bases, oximes, acetales, ketales, enolesters, oxazolidines, thiazolidines or combinations thereof.

The various steps recited above are generally known in the art. They include or rely on quantitative structure-activity relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 2000, 140(8): 813).

In a more preferred embodiment, the age related diseases are selected from the group consisting of fibrosis, inflammatory cardiomyopathy, heart hypertrophy, liver degeneration, skeletal muscle degeneration, chronic general inflammation.

As described hereinabove, Fibrosis generally relates to the formation of scar tissue in organs such as the liver or the heart. Inflammatory cardiomyopathy relates to an abnormal heart condition in which the heart is dilated (poor pumping power), restrictive (impaired ability of the heart to fill) or hypertrophic (enlarged heart). Heart hypertrophy is a disorder in which the heart muscle is so strong that it does not relax enough to fill the heart with blood and so has reduced pumping ability. Skeletal muscle degeneration generally refers to muscle wasting, sarcopenia as well as depletion of muscular tissues and fat stores. Chronic general inflammation generally relates to an infiltration of organs with inflammatory cells such as macrophages and neutrophile lymphocytes as well as increased levels of CRP and IL6. Emerging pathological evidence indicates that major chronic ageing-related diseases such as atherosclerosis, arthritis, dementia, osteoporosis and cardiovascular diseases are inflammation-related.

In another embodiment, the invention relates to a method for the identification of a compound useful in the treatment of age-related diseases or as a lead compound for the development of an agent for treating age-related diseases comprising the steps: (a) determining the level of Sirt7 transcript or protein in a cell wherein said cell comprises inducible Sirt7 DNA; (b) contacting said cell with a test compound; (c) determining the level of Sirt7 transcript or protein in said cell after contacting with the test compound; and (d) comparing the Sirt7 transcript or protein level determined in step (c) with the Sirt7 transcript or protein level determined in step (a) wherein an increase of Sirt7 transcript or protein level in step (c) as compared to step (a) indicates that the test compound is a compound useful in the treatment of age-related diseases or as a lead compound for the development of an agent for treating age-related diseases.

The term "inducible" in accordance with the present invention refers to an increase of Sirt7 transcript or protein levels in a cell, wherein this increase is a result of the induction of Sirt7 expression by the test compound. The "inducible Sirt7 DNA" may, for example, be the endogenous Sirt7 gene with it's naturally occurring Sirt7 promoter. Alternatively, the cell may be transfected with a construct comprising the Sirt7 gene and the naturally occurring promoter-active DNA fragments which are located in the 5'-flanking region of the gene and which are sufficient for transcriptional control of the Sirt7 gene. The level of Sirt7 transcript or protein may e.g. be undetectable before contacting the above-mentioned cell with the test compound and it may be clearly detectable after contacting the cell with the test compound in order to indicate a compound suitable for the treatment of age-related diseases or as a lead compound for the development of a compound for the treatment of age-related diseases. Alternatively, the above-mentioned cell may exhibit a detectable level of Sirt7 transcript or protein before contacting with the test compound and the level of Sirt7 transcript or protein may be higher after contacting the cell with the test compound. Preferably, the level of Sirt7 transcript or protein is for example at least 5, 10, 20, 30, 40 or 50% higher after contacting the cell with the test compound. More preferably, the level of Sirt7 transcript or protein is for example at least 100, 200, 300 or 400% higher after contacting the cell with the test compound. Most preferably, the level of Sirt7 transcript or protein is for example at least 500% higher after contacting the cell with the test compound.

As described hereinabove, besides the enhancement of enzymatic activity, also the increase of expression levels conferred by a compound may contribute to the age-suppressing activity of said compound. Accordingly, measurement of the transcript or protein level of Sirt7 is another approach to determine the readout of the above-described assay. The measurement of the protein level can be accomplished in several ways. Western blotting or polyacrylamide gel electrophoresis in conjunction with protein staining techniques such as, but not limited to, Coomassie Brilliant blue or silver-staining may be used. Also of use in protein quantification is the Agilent Bioanalyzer technique.

Techniques for the determination of the transcript level include, but are not limited to RT-PCR and its various modifications such as qRT-PCR (also referred to as Real Time RT-PCR). PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed for example in a 50 µl reaction mixture containing 5 µl of 10 x PCR buffer with 1.5 mM MgCl2, 200 µM of each deoxynucleoside triphosphate, 0.5 µl of each primer (10 µM), about 10 to 100ng of template DNA and 1 to 2.5 units of Taq Polymerase. The primers for the amplification may be labeled or be unlabeled. DNA amplification can be performed, e.g., with a model 2400 thermal cycler (Applied Biosystems, Foster City, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step at 55°C for 1 min as well as a final extension step at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, the person skilled in the art knows how to optimize PCR conditions for the amplification of specific nucleic acid molecules with primers of different length and/or composition or to scale down or increase the volume of the reaction mix. The "reverse transcriptase polymerase chain reaction" (RT-PCR) is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT Reaction can be performed, for example, in a 20µl reaction mix containing: 4 µl of 5x AMV-RT buffer, 2 µl of Oligo dT (100 µg/ml), 2µl of 10 mM dNTPs, 1µl total RNA, 10 Units of AMV reverse transcriptase, and H₂O to 20µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample. Any of the above mentioned reaction conditions may be scaled up according to the needs of the particular case.

Real-time PCR employs a specific probe, in the art also referred to as TaqMan probe, which has a reporter dye covalently attached at the 5' end and a quencher at the 3' end. After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor which was formerly quenched due to the close proximity to the 3' acceptor in the TaqMan probe sequence. Thereby, the process of amplification can be monitored directly and in real time, which permits a significantly more precise determination of expression levels than conventional end-point PCR. Also of use in Real time RT-PCR experiments is a DNA intercalating dye such as SybrGreen for monitoring the de novo synthesis of double stranded DNA molecules.

In a more preferred embodiment, said cell is a primary cell or primary cell line. Primary cells are cells which are directly obtained from an organism. Suitable primary cells are, for example, mouse embryonic fibroblasts, mouse primary hepatocytes, cardiomyocytes and neuronal cells as well as mouse muscle stem cells (satellite cells) and stable, immortalized cell lines derived thereof. Also within the scope of the present invention are mammalian cells such as Hela, HEK293, H9 and Jurkat cells, NIH3T3 and C127 cells, Cos 1, Cos 7 and CV1, QC1-3 cells, mouse L cells, mouse C2C12 cells and Chinese hamster ovary (CHO) cells.

In another more preferred embodiment said cell comprises a nucleic acid molecule encoding a protein having Sirt7 function fused to a reporter gene wherein said nucleic acid molecule (a) has the sequence of SEQ ID NO: 1,3,5 or 7; (b) encodes a protein having the sequence of SEQ ID NO: 2, 4, 6 or 8; (c) hybridizes under stringent conditions to the molecule of (a) or (b); or (d) has an identity on the nucleic acid level of at least 80% with the molecule of (a), (b) or (c).

The fusion to a reporter gene allows the indirect measurement of protein level by measuring the level of the reporter gene attached to the protein of interest. Examples of reporter genes include, but are not limited to, green fluorescent protein (GFP), yellow fluorescent protein (YFP), red fluorescent protein (RFP), cyan fluorescent protein (CFP), luciferase or β-galactosidase.

In another embodiment, the invention relates to a method of treating a subject suffering from an age-related disease by administering to said subject a pharmaceutical composition comprising (i) a nucleic acid molecule encoding a protein having Sirt7 function wherein said nucleic acid molecule (a) has the sequence of SEQ ID NO: 1, 3, 5 or 7; (b) encodes a protein having the sequence of SEQ ID NO: 2, 4, 6 or 8; (c) hybridizes under stringent conditions to the molecule of (a) or (b); or (d) has an identity on the nucleic acid level of at least 80% with the molecule of (a), (b) or (c); (ii) a vector comprising the nucleic acid molecule of (i); (iii) a host cell comprising the vector of (ii); or (iv) a protein encoded by the nucleic acid molecule of (i).
In a more preferred embodiment, the age related diseases are selected from the group consisting of fibrosis, inflammatory cardiomyopathy, heart hypertrophy, liver degeneration, skeletal muscle degeneration, chronic general inflammation.

As described hereinabove, Fibrosis generally relates to the formation of scar tissue in organs such as the liver or the heart. Inflammatory cardiomyopathy relates to an abnormal heart condition in which the heart is dilated (poor pumping power), restrictive (impaired ability of the heart to fill) or hypertrophic (enlarged heart). Heart hypertrophy is a disorder in which the heart muscle is so strong that it does not relax enough to fill the heart with blood and so has reduced pumping ability. Skeletal muscle degeneration generally refers to muscle wasting, sarcopenia as well as depletion of muscular tissues and fat stores. Chronic general inflammation generally relates to an infiltration of organs with inflammatory cells such as macrophages and neutrophile lymphocytes as well as increased levels of CRP and IL6. Emerging pathological evidence indicates that major chronic ageing-related diseases such as atherosclerosis, arthritis, dementia, osteoporosis and cardiovascular diseases are inflammation-related.

In a most preferred embodiment, the subject is a mammal, preferably a human.

The Figures show:

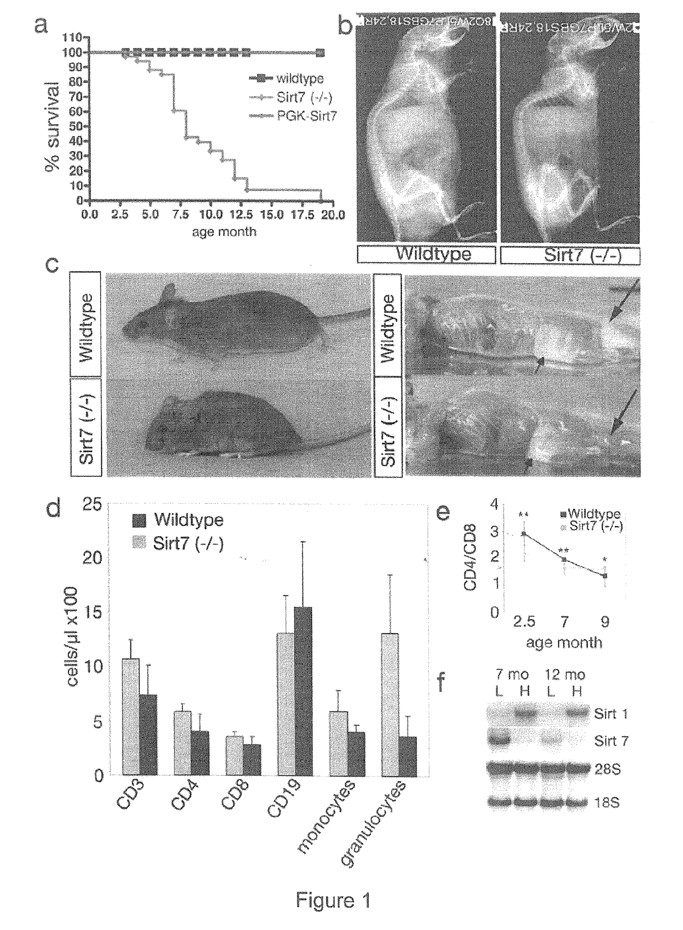

**Figure 1****:** a, Kaplan-Mayer survival curve showing a reduced life span of Sirt7 knockout mice. n = 98 (wild-type); n = 32 (knockout); n = 28 (overexpressing). Sirt7+/- animals were phenotypically indistinguishable from wild-type controls. b, X-ray pictures reveal a kyphosis of the vertebral column in knockout but not in wild-type mice. Both mice are 7 months old. At this age and older kyphosis was apparent in 80% of knockout animals. c, Left panel: General appearance of an 11 months old wild-type (left above) and a 7 months Sirt7 knockout (left below) mice. Right panel: Skinned wild-type and knockout mice to visualize differences in visceral (small arrows) and subcutaneous (big arrows) fat deposits. d, Increased numbers of T-lymphocytes (CD3+, CD4+ and CD8+), monocytes and granulocytes in the blood of Sirt7-/- mice as measured by FACS analysis. B-lymphocytes (CD19+) were slightly decreased while granulocytes were substantially increased in knockout mice. e, The ratio of CD4+ to CD8+ T-lymphocytes decreases prematurely in Sirt7 knockout mice. The age of mice is indicated in months. Data presented in d, e are mean ratios +/- s. d. from three different animals for each group (n = 3; P < 0.05). f, Northern blot analysis of Sirt1 and Sirt7 expression in liver (L) and heart (H) in wild-type mice at 7 and 12 months of age. Ribosomal RNA bands (28S and 18S) are shown for loading control.

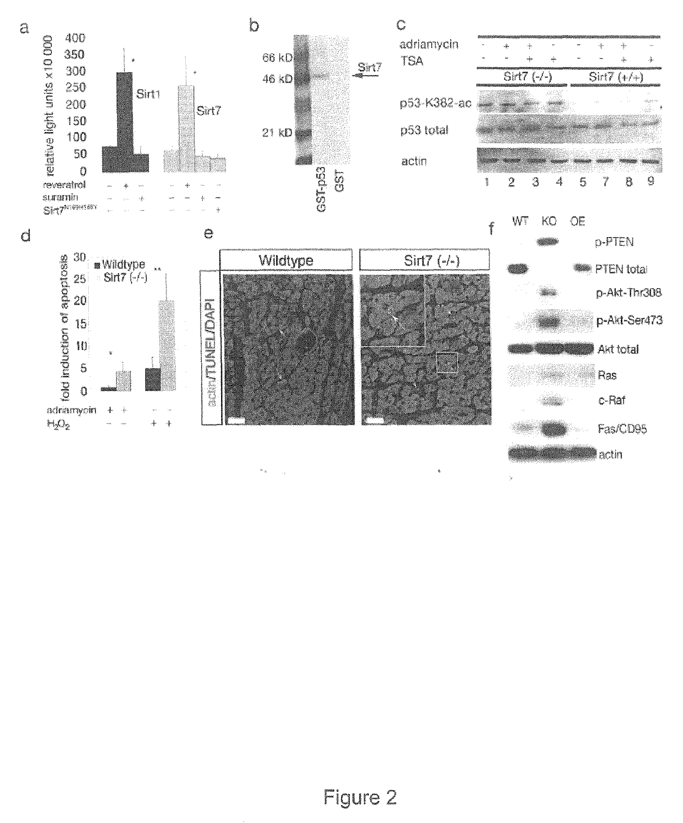

**Figure 2****:** Sirt7 deacetylates p53 and inhibits apoptosis. a, Sirt7 and Sirt1 deacetylate p53 peptides in vitro. The enzymatic activity of both enzymes was enhanced by resveratrol and inhibited by suramin. Sirt1 and Sirt7 inactivating mutations within the enzymatic domain strongly diminished the deacetylase activity. b, Sirt7 physically interacts with p53. Sirt7 in vitro translated 35S protein was retained on the p53-GST loaded matrix as demonstrated by SDS-gel analysis. No Sirt7 protein was bound to matrix covered with GST protein only. c, p53 is hyperacetylated in Sirt7 mutant cells. MEFs treated with adriamycin, TSA, or combination as indicated were investigated by Western blot analysis for total and acetylated p53. Actin was used as a loading control. d, The rate of apoptosis was estimated by ELISA in wild-type and Sirt7 deficient MEFs after treatment with adriamycin or H₂O₂. Apoptosis was calculated as a fold induction above the apoptotic rate of untreated cells. Values in a, and d represent means of three different experiments (performed in duplicate); error bars represent s. e. m. * P < 0.05; ** P < 0.001. e, Microscopic sections stained for TUNEL (red) reveal approximately 50% increase in apoptotic myocytes in Sirt7 knockout heart (inset). Sections were counterstained with sarcomeric α-actin (green) and DAPI (blue); bar = 30 µm. f, Western blot analysis of heart tissues of wild-type, Sirt7 knockout, and Sirt7 overexpressing animals. The band for total PTEN in the knockout sample is not visible due to the short exposure time.

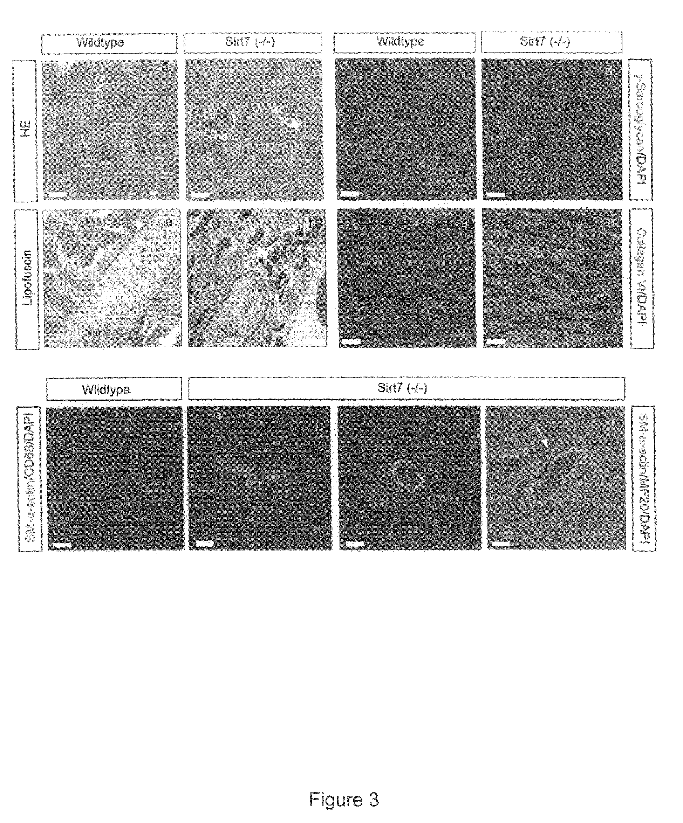

**Figure 3****:** Inflammatory cardiomyopathy in Sirt7 deficient animals. Representative tissue sections of the heart from wild-type (n = 3) and Sirt7 -/- (n = 4) animals are shown. a, b, Hematoxylin/eosin staining reveals mononuclear infiltrations in mutant myocardium. c, d, Staining for γ-sarcoglycan (green) and DAPI (blue) reveals hypertrophic cardiomyocytes, caliber variations, and disturbed tissue architecture in Sirt7 deficient hearts. e, f, Electron microscopy disclose lipofuscin deposits in knockout (arrows in f) but not in wild-type cardiomyocytes. g, h, Fibrosis of the myocardium of Sirt7-/- mice as revealed by collagenVI (green) accumulation. i - k, Sections were stained for CD68 (red) and smooth muscle α-actin (green) to visualize inflammatory lymphocytes and the re-expression of smooth muscle α-actin in Sirt7 knockout hearts, respectively. Lymphocytes were found at interstitial (j) and peri-vascular (k) locations. I, Smooth muscle α-actin expression (green) in endothelial vessel cells and in adjacent MF20 positive (red) cardiomyocyte; note the cross-striation in the stressed smooth muscle α-actin positive cardiomyocyte (arrow in I). Bars = 50 µm (a- d, g, h), 1 µm (e, f), 30 µm (i - I).

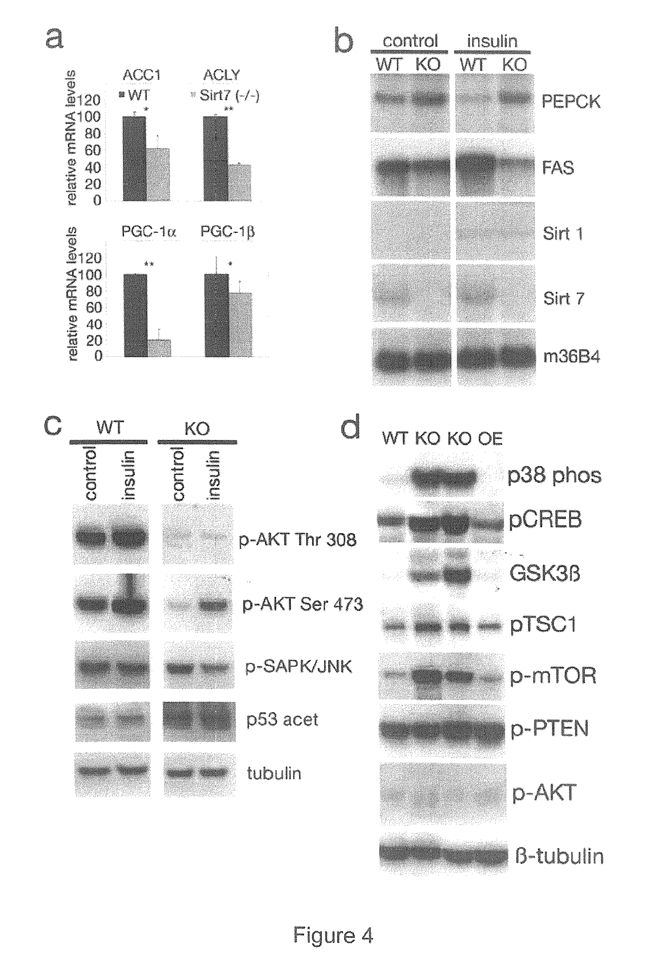

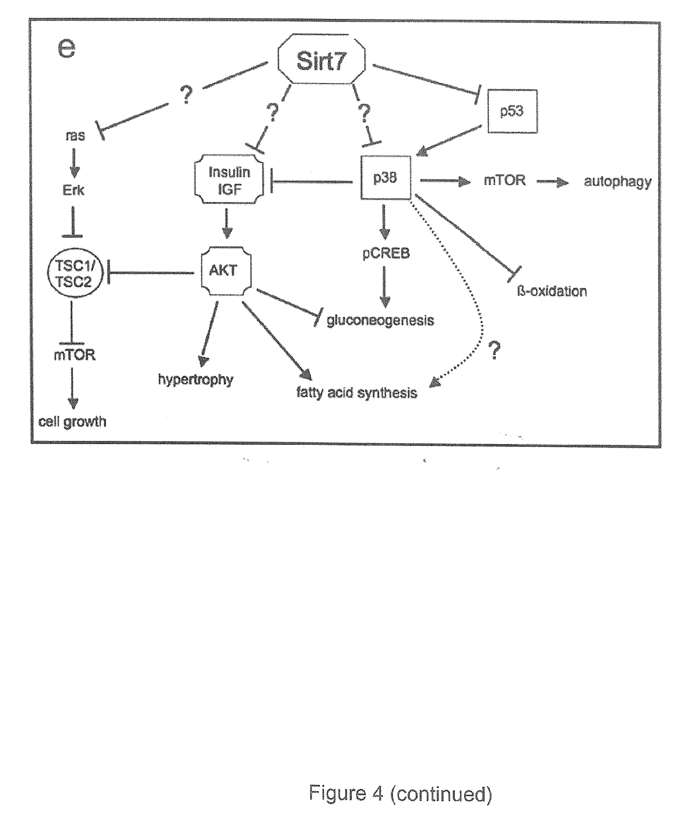

**Figure 4****:** Dysregulation of gluconeogenesis and fat synthesis, lack of Akt phosphorylation, and increased stress signaling in Sirt7 deficient cells. a, Quantitative RT-PCR analysis of acetylcarboxylase (ACC1) and ATP-citrate lyase (ACLY) and PGC-1α and PGC-1β in the liver. The relative mRNA levels represent means from three different animals per group and were calculated as a difference of expression between wild-type and knockout in percent. * P< 0.001; ** P < 0.005; *** P < 0.2. b, Northern blot analysis of expression of phosphoenolopyruvate kinase (PEPCK), fat synthase (FAS), Sirt1 and Sirt7 in cultivated primary hepatocytes from wild-type (WT) and Sirt7-/- mice with and without 100 mM insulin. Ribosomal protein m36B4 was used as a loading control. c, Western blot analysis of protein expression in primary hepatocytes from wild-type (WT) and Sirt7-/- mice treated as in (b). d, Western blot analysis of protein expression in livers of wild-type (WT), two different Sirt7 knockout (KO) and Sirt7 overexpressing mice. e, Summary of changes in cellular signaling in Sirt7 knockout tissues.

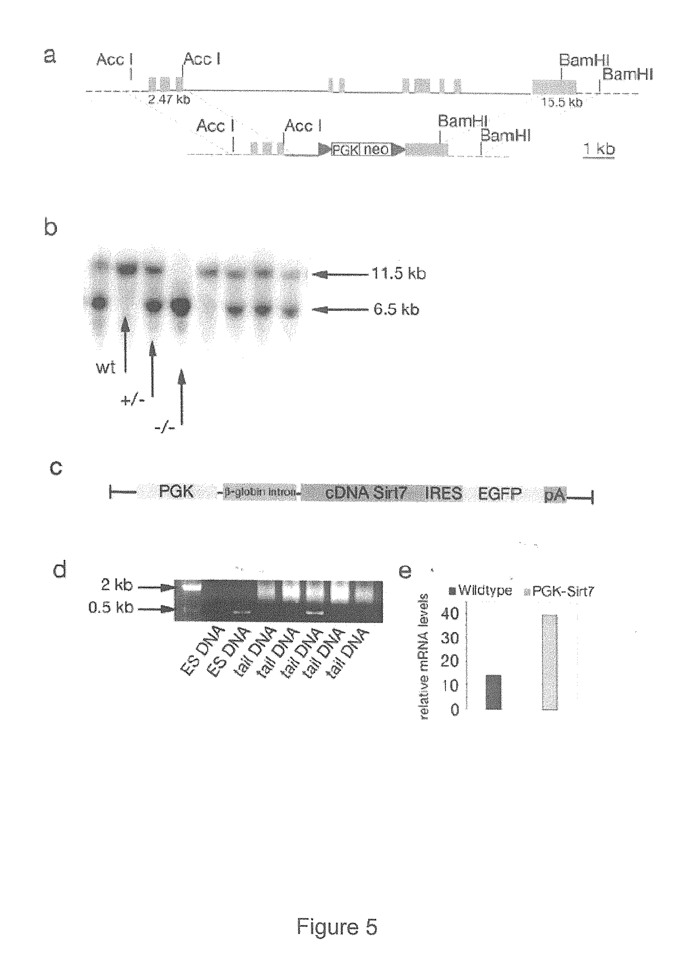

**Figure 5****:** Generation of Sirt7 deficient and Sirt7 overexpressing animals. a, Representation of genomic Sirt7 regions used for construction of the targeting vector for homologous recombination in ES cells. b, Southern blot analysis of wild type, Sirt7 heterozygous and Sirt7 deficient animals; the 11.5 kb EcoRV band represents the wild-type allele, which is converted to 6.5 kb after homologous recombination. c, The construct used for pro-nucleus injection contains the PGK promoter, β-globin intron sequences and the Sirt7 full length cDNA. It also contains an IRES-EGFP cassette and a polyA signal at its 3'-end. d, Southern blot analysis showing an additional 0.5 kb band indicating a successful genomic integration of Sirt7 expressing plasmid in control embryonic stem cells and Sirt7 transgenic tail DNA. e, Quantitative RT-PCR analysis showing a 2.5 fold increase in Sirt7 expression in tail tissues of Sirt7 overexpressing mice.

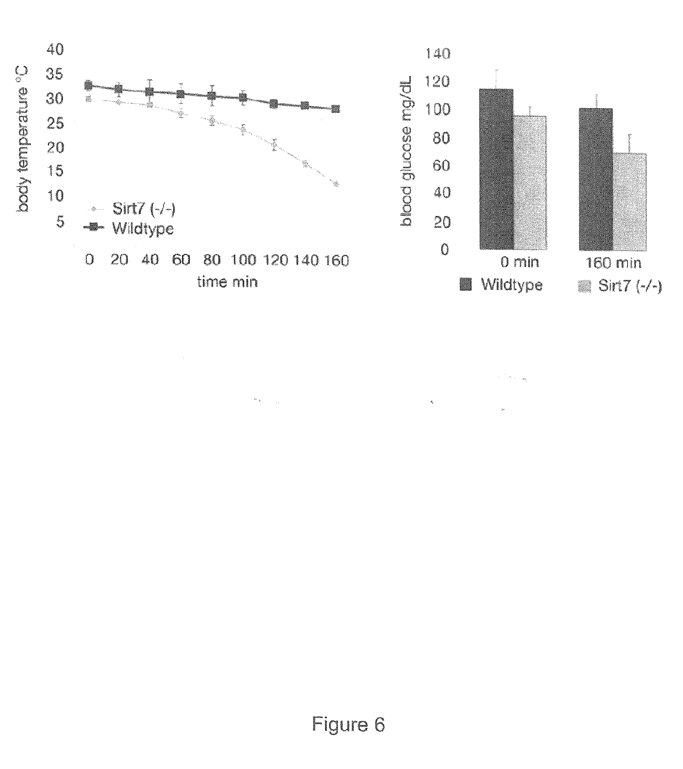

**Figure 6****:** Sirt7-/- mice are hyper-sensitive to cold and starvation. a, Body temperature was estimated using a TAIOTA-F20 implant and plotted against time in minutes. b, Blood glucose was estimated at two time points as indicated. Three animals for each group were tested; * P < 0.01; ** P < 0.001.

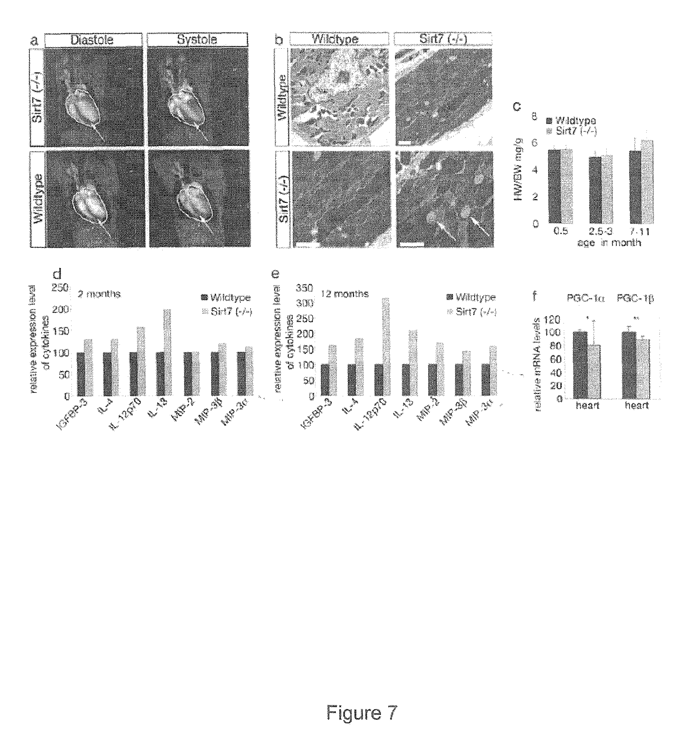

**Figure 7****:** Heart hypertrophy and ultrastructure of the myocardium of Sirt7 mutant animals. a, MRI analysis: four chamber views of hearts of knockout and wild type mice in diastole and systole. Representative male animals at the age of five months are shown. Arrows mark the thickened septum on the knockout heart as compared with wild type. The mutant heart is also enlarged relative to the body size, as indicated by the superimposed knockout heart contour (thin white line) on the wild type heart. b, EM images of heart tissue sections of wild-type and Sirt7 knockout mice. The lower panels represent higher magnification of upper pictures for better visualization of a pycnotic, apoptotic nucleus (nuc) and vacuoles (arrows) present in the knockout myocardium. c, The heart/body ratio (HW/BW) was increased in older Sirt7 knockout mice (7 - 11 months) as compared with younger animals (2.5 - 3 months of age). No heart hypertrophy or diminished body weight were observed shortly after birth (two weeks of age). Error bars represent s. e. m. * P < 0.05. d, e, An increased cytokine production in Sirt7 mutant myocardium. 500µg of wild-type and Sirt7 mutant heart protein extracts of two (c) and 12 (d) month old animals were reacted with Mouse Cytokine Antibody Array III, Ray Biotech, Inc. The difference between hybridization intensity of wild-type (100%) and knockout was shown in the diagrams. f, Quantitative RT-PCR analysis of expression of transcription factors PGC-1α and PGC-1β in the heart. The relative mRNA levels are means of measurements from three different animals per group and calculated as a difference in expression between wild type and knockout in percent. * P< 0.005; ** P < 0.001.

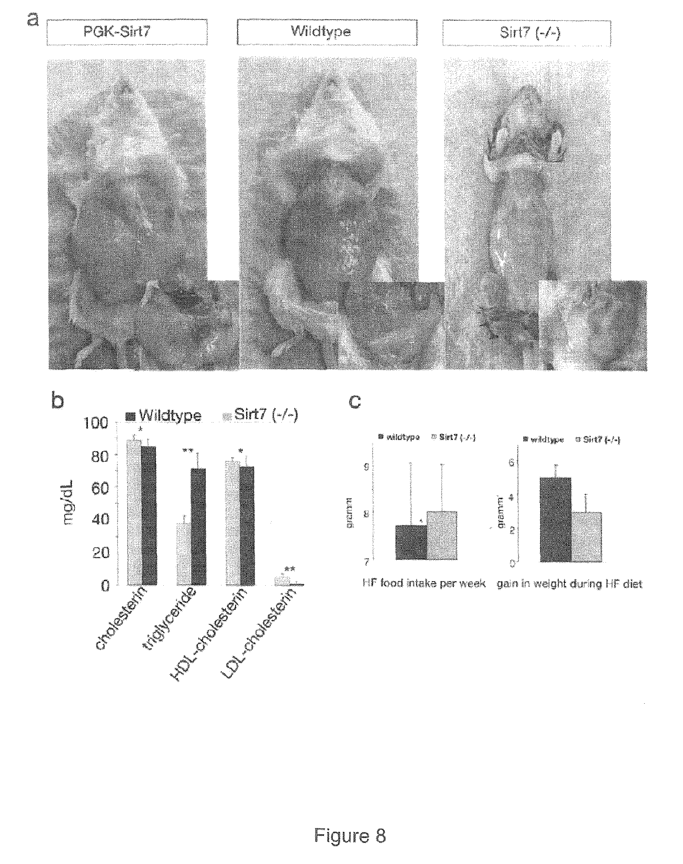

**Figure 8****:** Fat accumulation, high-fat diet resistance and elevated triglycerides levels in Sirt7 deficient mice. a, Mice were dissected to visualize differences in the amount of visceral fat between wild type, Sirt7 overexpressing (PGK-Sirt7) and Sirt7 knockout individuals. Insets show enlarged views on fat tissue from the lower left abdominal region. b, Total cholesterol, triglycerides, HDL-, and LDL-concentrations are mean values of three individuals per group; * P< 0.2; ** P < 0.005. c, Wilde-type and Sirt7-/mice were fed a high-fat diet for a time period of 5 months. The food intake per week and the total gain in weight are shown. Each group consisted of 5 animals.

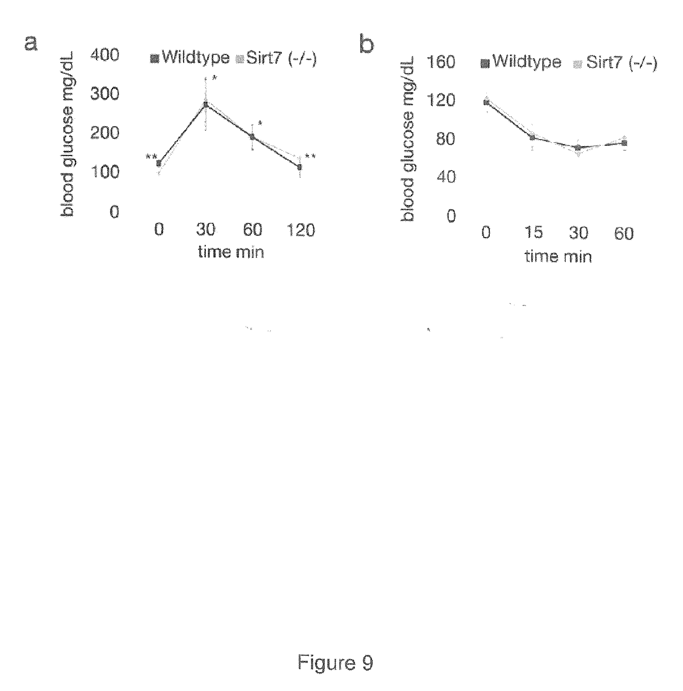

Figure 9: Glucose and insulin tolerance is maintained in Sirt7 knockout mice. a, b, Glucose and insulin tolerance tests respectively. Mean values of six (a) or three (b) different animals per group are presented; * P < 0.3; ** P< 0.03 (a); P < 0.2 (b).

The Examples illustrate the invention:

### Materials and Methods

### Mice

Sirt7 knockout and overexpressing mice were generated as described in Example 1. Knockout animals were back-crossed to C57BI/6 mouse strain and the transgenics to the ICR strain for at least four generations to ensure the same genetic background for control and mutant animals. No phenotypic differences were present in the first generation animals on mixed C57BI/6; 129Sv background, which were used for some initial analyses. For all studies male littermates, which were kept under the same conditions, were used.

### Cell culture

Mouse embryonic fibroblasts (MEFs) and primary liver hepatocytes were cultured under standard conditions as described in Braun et al. Cell 1992, 71: 369. Primary hepatocytes were isolated from perfused livers according to Seglen, P.O. Methods Cell Biol. 1976, 13: 29.

### Deacetylation assay

Deacetylation activity of recombinant mouse Sirt1 and Sirt7 protein was estimated using acetylated p53 peptides (p53-382/diAc) and Sirt1 Fluorometric Drug Discovery Kit - AK-555, BIOMOL according to manufacturers protocol.

### Apoptosis

Apoptotic cardiomyocytes on heart tissue sections were visualized using TUNEL staining and analyzed by confocal laser scanning microscopy. Apoptosis in cultured MEFs was measured by Cell Death Detection ELISA (Roche).

### MRI

Magnetic Resonance Imaging was performed under volatile isoflurane (2%) anesthesia with a 7.05 T (Bruker) MR scanner equipped with a 300mT/m gradient system, which works 300.51 MHz for isotope 1 H. A 2.6 cm usable diameter quadrature low-pass birdcage coil was constructed in-house (Wagner et al. NMR Biomed. 2004, 17: 21) and used in all experiments. MRI experiments were performed by applying an ECG-triggered gradient-echo sequence with the following imaging parameters: echo time (TE) = 2.9ms; repetition time (TR) = 128.5ms; field of view (FOV) = 4.00 x 4.00cm2; acquisition matrix = 129 x 256; maximal in-plane resolution = 156 x 312 mm2; slice thickness = 1.0mm. One four-chamber view was obtained for each heart.

### FACS

Blood cells were characterized by standard flow cytometry as described (Schulze et al. Genes Dev. 2005, 19: 1787) using PE- or FITC-conjugated antibodies against CD3, CD4, CD8, CD11b, CD19, and CD45. Data collected from >10,000 cells were expressed as the percentage of positive cells per total gated cells. Raw data were analyzed using the CellQuest pro software (BD Inc.).

### Western blotting

Whole heart tissue or cell culture lysates were used. Cultures were washed three times with HBSS and lysed at the indicated time in Laemmli buffer containing 0.2M PMSF, 1M Na₃VO₄, 1M NaF, 1 mg/ml Aprotinin and Leupeptin (all from Sigma). 10 mg of protein lysates were separated on 4%-12% SDS-PAGE gradient gels and transferred onto nitrocellulose membranes (invitrogen Life technologies, Groningen, The Netherlands). Immunoreactive proteins were visualized with corresponding HRP-conjugated secondary antibodies on Hyperfilm (GE Healthcare) using the SuperSignal West Pico or West Femto detection solutions (Perbio Science). Blots were scanned using a STORM 860 (Molecular Dynamics, Freiburg, Germany) and analyzed using ImageQuant software (Molecular Dynamics). Antibodies directed against p-PTEN, total PTEN, pAkt, total Akt, Ras, pan-actin, Fas/CD95 were from NEB (Cell Signalling) and c-Raf was from Becton Dickinson.

### IHC

Tissues for immunochistochemistry were fixed and either embedded in paraffin or shock-frozen for cryosectioning, or processed for electron microscopy applying standard procedures (Oustanina et al. EMBO J 2004, 23: 3430). The following antibodies were used: MF20, anti-γ-Sarcoglycan, anti-CollagenVI (Rockland), anti-CD68 (Dako), anti-SM-α-actin-antibody (Cymbus Biotechnology). Secondary antibodies were coupled with Alexa 596 (red), Alexa 350 (blue), and Alexa 488 (green) and used according to the manufacturer's instructions (Molecular Probes). Nuclei were visualized using a 30µmolar DAPI solution (Molecular probes).

### Quantitative RT-PCR

RNA was isolated using established procedures that have been described previously (Braun et al. Cell 1992, 71: 369). Quantitative RT PCR was achieved by cDNA amplification in the presence of the DNA-binding dye SYBR Greenl as described previously (Neuhaus et al. Mol. Cell Biol. 2003, 23: 6037). The following primer pairs were used:
ACC1: 5'-CAGGCCGGCCAGGTTTG-3' (SEQ ID NO: 9)
   and 5'-TCCATGTGCCGAGGGTTGAT-3' (SEQ ID NO: 10)
ACLY: 5'-GAGGTGGCCCCAACTATCAAGAGG-3' (SEQ ID NO: 11)
   and 5'-CCCGCTGGCATTAAGGAGGAAGTT-3' (SEQ ID NO: 12)
PGC-1a: 5'-TACAATGAATGCAGCGGTCTTAGC-3' (SEQ ID NO: 13)
   and 5'-GAGGAGGGTCATCGTTTGTGGT-3' (SEQ ID NO: 14)
PGC-1b: 5'-GCCCTGGAAAGCCCCTGTGAGAGT-3' (SEQ ID NO: 15)
   and 5'-GTGTGGTGGGTGGCGTGAGTCCTG-3' (SEQ ID NO: 16)
Sirt7: 5'-CCCCGGACCGCCATCTCAG-3' (SEQ ID NO: 17)
   and 5'-ATCTCCAGGCCCAGTTCATTCAT-3' (SEQ ID NO: 18)

The relative amount of mRNA was calculated using the formula: [SQ_{target}/SQ_{HPRT}]. Relative values obtained for mutant and wild type samples were compared to each other.

### Blood parameter measurements

For glucose and insulin tolerance measurements mice were set under fasting conditions for 4h. Samples of approximately 300µl were collected at time points indicated in Figure 7. Glucose concentration was estimated by Accu-Check Sensor Comfort (Roche). For blood lipid estimation approximately 225µl of blood (6-8 drops plus one full capillary tube) were collected from each mouse via retro-orbital bleed using heparin-coated hematocrit tubes. A minimum of 100µl of blood plasma was collected and used for lipid estimation using Beckman Coulter Synchron CX5 (Chemistry Analyzer) according to manufacturers protocol.

### Example 1: Generation of Sirt7 knockout and overexpressing mouse strains.

The targeting vector to obtain Sirt7 knockout mice was constructed by insertion of a 2.5kb Accl fragment derived from the 5'-region of the Sirt7 gene comprising the first three exons into the vector pK11 in front of a neomycin-resistance cassette, flanked by frt recognition sites for Flp-recombinase. A 15.5kb Sall-BamHl fragment from the 3'-region of the Sirt7 gene was inserted behind the selection cassette to generate a 3'-homology fragment (Fig. 5a). The recombination vector replaced the entire conserved region, were the enzymatic activity resides (North and Verdin, Genome Biology 2004, 5: 224) by the neomycin gene. Electroporation and selection of J1 ES cells were done as described previously (Kruger et al. EMBO J 2004, 23: 4353). To identify correctly targeted clones a 5' external probe (PCR-generated 700 bp fragment) was used to detect a 11.5kbp EcoRV fragment in the wild-type allele and a 6.5kb fragment in the mutant allele by Southern blot hybridization (Fig. 5b). Mice overexpressing Sirt7 were generated by pro-nucleus injection of a PGK-Sirt7-IRES-EGFP fragment (Fig. 5c). Southern blot analysis was used to confirm successful genomic integration of Sirt7 expressing plasmid in control embryonic stem cells and Sirt7 transgenic tail DNA (Fig. 5d). Increase in Sirt7 expression was confirmed using quantitative RT-PCR analysis (Fig. 5e).
The Sirt7 -/- mice had a reduced lifespan, most animals died between 10 and 13 months without any apparent cause of death. In contrast Sirt7 overexpressing animals showed no decrease in the life span (Fig. 1a). Furthermore, Sirt7 deficient mutants exhibited several signs of premature aging beginning at five months of age: mutant mice were smaller than their littermates, they developed kyphosis, and were characterized by a reduced amount of visceral and subcutaneous fat tissue (Fig. 1b, c). In addition, Sirt7 mutants suffered from chronic inflammation indicated by increased numbers of granulocytes and T-lymphocytes in the blood (Fig. 1d) and also by invasion of several organs by inflammatory cells (Fig. 3, Fig. 7d and e). Despite a general increase in T-lymphocytes, the CD4/CD8 ratio was reduced prematurely at the age of 2.5 months (Fig. 1e) indicating the presence of aged T-lymphocytes subsets. Finally, Sirt7 expression dropped in several tissues, particularly in the liver of older animals concurring with a function to prevent aging (Fig. 1f).

### Example 2: Sirt7 deacetylates p53 in vitro

p53 acetylated peptides were efficiently deacetylated by Sirt7 in vitro and Sirt7 dependent p53 deacetylation was stimulated by a polyphenolic compound, resveratrol, which has been previously thought to specifically induce the enzymatic activity of Sirt12 (Fig. 2a). In another assay it was also demonstrated that Sirt7 can physically interact with p53 (Fig. 2b). In agreement with the ability of Sirt7 to deacetylate and inactivate p53, higher levels of acetylated p53 in mouse embryonic fibroblasts (MEFs) and in primary hepatocytes of Sirt7 mutant mice (Fig. 2c and 4c) were detected. Furthermore, Sirt7 knockout MEFs were found to be more susceptible to genotoxic and oxidative stress (Fig. 2d).

### Example 3: Sirt7 mutants are sensitive to environmental stressors

Sirt7 mutants are very sensitive to combined cold and starvation stress. Sirt7 deficient mice were not able to keep a constant body temperature and normal blood glucose concentrations under cold stress (4°C) preceded by over night fasting and died during the first three hours of cold exposure when the body temperature dropped below 20°C and the blood glucose concentration below 70 mg/dl (Fig. 6)

### Example 4: Inflammatory cardiomyopathy in Sirt7-deficient animals

Sirt7-/- mice suffer from hypertrophic, inflammatory cardiomyopathy. Staining with γ-sarcoglycan indicated an enlargement of the majority of cardiomyocytes with strong caliber variations reflecting ongoing cardiac remodelling (Fig. 3c, d). Lipofuscin, which is an intralysosomal, polymeric, undegradable material that accumulates in aged tissues, was found as inclusions in Sirt7 deficient cardiomyocytes, while wild-type cardiomyocytes at this age were devoid of any lipofuscin deposit (Fig. 3e, f). The degenerative changes in Sirt7 mutant hearts did also result in a strong fibrosis as indicated by collagen VI accumulation and in the re-expression of smooth muscle α-actin in cardiomyocytes (Fig. 3h, l). Inflammmatory infiltrations were confirmed at interstitial and vascular locations by immunostaining with a CD68 antibody specific for inflammatory lymphocytes (Fig. 3j, k).

### Example 5: Influence of Sirt7 on the Akt-signalling pathway and on fat synthesis

Sirt7-/- mice developed an increased heart/body ratio by 2.5 months of age, inflammation and a higher rate of apoptosis (Fig. 2e, Fig. 3 a, b, and Fig. 7c). Also observed were increased levels of (activated) phosphorylated Akt in Sirt7-/- hearts along with an increase of ras and c-raf proteins (Fig. 2f), which might be involved in the activation of Akt. The Akt inhibitor and tumor suppressor molecule PTEN was reduced in mutant hearts as indicated by a strong reduction of the PTEN protein and an increase in (inactivated) phosphorylated PTEN21 (Fig. 2f).
Sirt7-/- mice showed a smaller size and leanness, which became apparent at around five months of age (Fig. 1c). Since the food consumption of Sirt7-/- mice was even slightly increased the leanness of the animals cannot be attributed to a restricted calorie intake but to a metabolic dysfunction. Interestingly, the blood cholesterol levels were normal in Sirt7 knockouts, but blood triglycerides levels were dramatically decreased. This was further supported by the increased accumulation of fat in Sirt7 overexpressing animals (Fig. 8). Analysis of PGC-1α expression, which is a critical regulator of metabolic homeostasis that is also positively regulated by Sirt1 via deacetylation, revealed a significant reduction of the expression of both, PGC-1α and PGC-1β, regulators in the liver of Sirt7-/- animals along with a decreased expression of key enzymes of fatty acid synthesis such as ACC and ACLY (Fig. 4a). In contrast, only a modest reduction of PGC-1α and PGC-1β expression was observed in the heart (Fig. 7f). Experiments with primary hepatocyte cultures revealed additional metabolic malfunctions, Most striking, the activation of a critical enzyme of gluconeogenesis, PEPCK, and inactivation of fatty acid synthase, FAS, by insulin were severely impaired in Sirt7-/- hepatocytes (Fig. 4b). Surprisingly, without Sirt7, insulin was unable to activate its primary signaling targets in hepatocytes as indicated by low levels of Akt- and SAPK/JNK phosphorylation (Fig. 4c). The impaired hepatic insulin signaling might be at least partly explained by a hyperactivation of p38 stress kinase in Sirt7 mutant livers (Fig. 4d). Despite the insulin resistance of cultured hepatocytes blood glucose homeostasis and insulin sensitivity were normal in knockout mice (Fig. 9).
Whereas the applicants do not wish to be bound by any scientific theory, the model as outlined in Figure 4e summarizes the changes in cellular signalling in Sirt7 knockout tissues. AKT is differentially regulated in the myocardium and in the liver. In the wild-type myocardium Sirt7 prevents over-activation of the AKT-signaling that might lead to hypertrophy in Sirt7 deficient myocardium. Cellular growth and proliferation might also be held in check by Sirt7 through downregulation of ras signaling, which otherwise will activate mTOR by inhibiting the mTOR-inhibitors, TSC1/TSC2. In the liver Sirt7 improves insulin-mediated inhibition of gluconeogenesis and activation of fat synthesis. This dysregulation of insulin signaling in Sirt7 deficient hepatocytes seems to be caused by an increase of p38 phosphorylation. The p38 hyperactivation was liver-specific and was not observed in Sirt7 knockout hearts.

## Claims

1. A pharmaceutical composition comprising
(i) a nucleic acid molecule encoding a protein having Sirt7 function
wherein said nucleic acid molecule
(a) has the sequence of SEQ ID NO: 1, 3, 5 or 7;
(b) encodes a protein having the sequence of SEQ ID NO: 2, 4, 6 or 8;
(c) hybridizes under stringent conditions to the molecule of (a) or (b); or
(d) has an identity on the nucleic acid level of at least 80% with the molecule of (a), (b) or (c);
(ii) a vector comprising the nucleic acid molecule of (i);
(iii) a host cell comprising the vector of (ii); or
(iv) a protein encoded by the nucleic acid molecule of (i).

2. Use of
(i) a nucleic acid molecule encoding a protein having Sirt7 function, wherein said nucleic acid molecule
(a) has the sequence of SEQ ID NO: 1, 3, 5 or 7;
(b) encodes a protein having the sequence of SEQ ID NO: 2, 4, 6 or 8;
(c) hybridizes under stringent conditions to the molecule of (a) or (b); or
(d) has an identity on the nucleic acid level of at least 80% with the molecule of (a), (b) or (c);
(ii) a vector comprising the nucleic acid molecule of (i);
(iii) a host cell comprising the vector of (ii); or
(iv) a protein encoded by the nucleic acid molecule of (i)
for the manufacture of a pharmaceutical composition for treating age-related diseases.

3. The use of claim 2 wherein the age-related diseases are selected from the group consisting of fibrosis, inflammatory cardiomyopathy, heart hypertrophy, liver and skeletal muscle degeneration, chronic general inflammation.

4. A method for the identification of a compound useful in the treatment of age-related diseases or as a lead compound for the development of an agent for treating age-related diseases comprising the steps:
(a) contacting Sirt7 protein with a test compound and an acetylated Sirt7 substrate; and
(b) determining the level of the deacetylated Sirt7 substrate and/or the level of the acetylated Sirt7 substrate before contacting the protein with the test compound and after contacting the protein with the test compound wherein a reduced level of acetylated Sirt7 substrate or an increased level of deacetylated Sirt7 substrate after contacting the protein with the test compound as compared to the level before contacting the protein with the test compound indicates that the test compound is a compound useful in the treatment of age-related diseases or as a lead compound for the development of an agent for treating age-related diseases.

5. The method according to claim 4 wherein the Sirt7 substrate is p53.

6. A method for the identification of a compound useful in the treatment of age-related diseases or as a lead compound for the development of an agent for treating age-related diseases comprising the steps:
(a) determining the level of Sirt7 transcript or protein in a cell wherein said cell comprises inducible Sirt7 DNA;
(b) contacting said cell with a test compound;
(c) determining the level of Sirt7 transcript or protein in said cell after contacting with the test compound; and
(d) comparing the Sirt7 transcript or protein level determined in step (c) with the Sirt7 transcript or protein level determined in step (a) wherein an increase of Sirt7 transcript or protein level in step (c) as compared to step (a) indicates that the test compound is a compound useful in the treatment of age-related diseases or as a lead compound for the development of an agent for treating age-related diseases.

7. The method according to claim 6 wherein said cell is a primary cell or primary cell line.

8. The method according to claim 6 or 7 wherein said cell comprises the nucleic acid molecule as defined in claim 1(i) fused to a reporter gene.

9. The method according to any one of claims 4 to 8 wherein the age-related diseases are selected from the group consisting of fibrosis, inflammatory cardiomyopathy, heart hypertrophy, liver and skeletal muscle degeneration, chronic general inflammation.


SEQUENCE LISTING

<110> Max-Planck-Gesellschaft zur Förderung der Wissenschaften
e.V.

<120> Use of Sirt7 for treating age-related diseases

<130> M2522 EP

<140> 07 10 2070.5
<141> 2007-02-09

<160> 18

<170> PatentIn version 3.3

<210> 1
<211> 1711
<212> DNA
<213> mus musculus

<400> 1
gcagccggtg gcggtctgag ccgctcggag cgcaaagctg ctgagcgggt ccggaggctg 60

cgggaggagc agcagcggga gcgcctccgc caggtgtcac gcatcctgag gaaggcggct 120

gcagagcgca gcgcggagga gggccggctc ttggccgaga gcgaggatct ggtgaccgag 180

ctgcagggtc gaagtcggcg gcgtgagggc ctcaagcgcc gccaggagga ggtgtgtgat 240

gacccggagg agctgcggag gaaggtccgc gaactggccg gagctgtccg aagtgccagg 300

cacttggttg tctacacggg cgctggaatc agcacagcag cttctatccc agattatcgg 360

ggtcctaatg gagtatggac actgcttcag aaaggaaggc ctgtgagtgc tgccgaccta 420

agcgaagcgg agcctaccct cacccacatg agcatcaccc gtttgcatga gcaaaagctg 480

gtgcaacacg tggtgtctca gaactgtgat gggctccacc tgcggagtgg gttgccccgg 540

accgccatct cagagctcca tgggaatatg tatattgaag tctgcacctc ctgcatccct 600

aacagagagt atgtacgagt gtttgatgtg actgagcgta ctgcccttca ccgacacctg 660

acaggccgga cctgccacaa gtgcgggacc cagcttcggg ataccattgt gcactttggg 720

gagaggggga cattagggca gcctctgaac tgggaggcag cgaccgaggc tgctagcaaa 780

gcagacacaa tcctgtgttt agggtccagc ttgaaggtac taaagaaata tccccgcctc 840

tggtgcatga cgaagcctcc aagccgtcga cccaaactct acatcgtgaa cctgcagtgg 900

accccgaagg atgactgggc tgccctgaaa ctccatggga agtgtgatga tgtcatgcaa 960

ctcctcatga atgaactggg cctggagatt cctgtctaca accggtggca ggatccaatc 1020

ttctccttgg cgacccccct ccgtgctggt gaagaaggca gccacagtag gaagtcacta 1080

tgcagaagca gagaagaagc cccacctggg gaccagagtg acccccttgc ctcagctccc 1140

cctatcctag gaggctggtt tggcaggggt tgtgccaagc gtgcaaaaag gaagaaagtg 1200

gcatagcccg gagttgacaa agaacagttg gcactttgca tatggcttga cccttgttca 1260

ggggctccgc ccgtgtcccg taaggcgaga gtgccccctc cggctctagt gggtgccggc 1320

ttcgcggtga cggtcgtctt tagccatttg tccttgtgga aaggctctct tgcactgctg 1380

tgactgcccc gctatgggct tgatcttcaa ggacacctcc ctcctgttca cccctgtaca 1440

aaggtggcag caaggcctct gtgagaaagc tccggccctt cccaggcctg gccagcagga 1500

gagtccacca ctgccttacc tcactctcca ggctagtctc agggcctccc tctttctact 1560

ccttatcacg tacaagtgtt cactttatag aagccttttt ctgtattggg tatgcggcac 1620

agggagaatc taactaagcc tccagtctga gcaagtctta ttaaacatct ctcaattgca 1680

aaaaaaaaaa aaaaaaaaaa aaaaaaaaaa a 1711

<210> 2
<211> 402
<212> PRT
<213> mus musculus

<400> 2

Met Ala Ala Gly Gly Gly Leu Ser Arg Ser Glu Arg Lys Ala Ala Glu
1               5                   10                  15

Arg Val Arg Arg Leu Arg Glu Glu Gln Gln Arg Glu Arg Leu Arg Gln
            20                  25                  30

Val Ser Arg Ile Leu Arg Lys Ala Ala Ala Glu Arg Ser Ala Glu Glu
        35                  40                  45

Gly Arg Leu Leu Ala Glu Ser Glu Asp Leu Val Thr Glu Leu Gln Gly
    50                  55                  60

Arg Ser Arg Arg Arg Glu Gly Leu Lys Arg Arg Gln Glu Glu Val Cys
65                  70                  75                  80

Asp Asp Pro Glu Glu Leu Arg Arg Lys Val Arg Glu Leu Ala Gly Ala
                85                  90                  95

Val Arg Ser Ala Arg His Leu Val Val Tyr Thr Gly Ala Gly Ile Ser
            100                 105                 110

Thr Ala Ala Ser Ile Pro Asp Tyr Arg Gly Pro Asn Gly Val Trp Thr
        115                 120                 125

Leu Leu Gln Lys Gly Arg Pro Val Ser Ala Ala Asp Leu Ser Glu Ala
    130                 135                 140

Glu Pro Thr Leu Thr His Met Ser Ile Thr Arg Leu His Glu Gln Lys
145                 150                 155                 160

Leu Val Gln His Val Val Ser Gln Asn Cys Asp Gly Leu His Leu Arg
                165                 170                 175

Ser Gly Leu Pro Arg Thr Ala Ile Ser Glu Leu His Gly Asn Met Tyr
            180                 185                 190

Ile Glu Val Cys Thr Ser Cys Ile Pro Asn Arg Glu Tyr Val Arg Val
        195                 200                 205

Phe Asp Val Thr Glu Arg Thr Ala Leu His Arg His Leu Thr Gly Arg
    210                 215                 220

Thr Cys His Lys Cys Gly Thr Gln Leu Arg Asp Thr Ile Val His Phe
225                 230                 235                 240

Gly Glu Arg Gly Thr Leu Gly Gln Pro Leu Asn Trp Glu Ala Ala Thr
                245                 250                 255

Glu Ala Ala Ser Lys Ala Asp Thr Ile Leu Cys Leu Gly Ser Ser Leu
            260                 265                 270

Lys Val Leu Lys Lys Tyr Pro Arg Leu Trp Cys Met Thr Lys Pro Pro
        275                 280                 285

Ser Arg Arg Pro Lys Leu Tyr Ile Val Asn Leu Gln Trp Thr Pro Lys
    290                 295                 300

Asp Asp Trp Ala Ala Leu Lys Leu His Gly Lys Cys Asp Asp Val Met
305                 310                 315                 320

Gln Leu Leu Met Asn Glu Leu Gly Leu Glu Ile Pro Val Tyr Asn Arg
                325                 330                 335

Trp Gln Asp Pro Ile Phe Ser Leu Ala Thr Pro Leu Arg Ala Gly Glu
            340                 345                 350

Glu Gly Ser His Ser Arg Lys Ser Leu Cys Arg Ser Arg Glu Glu Ala
        355                 360                 365

Pro Pro Gly Asp Gln Ser Asp Pro Leu Ala Ser Ala Pro Pro Ile Leu
    370                 375                 380

Gly Gly Trp Phe Gly Arg Gly Cys Ala Lys Arg Ala Lys Arg Lys Lys
385                 390                 395                 400

Val Ala

<210> 3
<211> 1712
<212> DNA
<213> mus musculus

<400> 3
agccggtggc ggtctgagcc gctcggagcg caaagctgct gagcgggtcc ggaggctgcg 60

ggaggagcag cagcgggagc gcctccgcca ggtgtcacgc atcctgagga aggcggctgc 120

agagcgcagc gcggaggagg gccggctctt ggccgagagc gaggatctgg tgaccgagct 180

gcagggtcga agtcggcggc gtgagggcct caagcgccgc caggaggagg tgtgtgatga 240

cccggaggag ctgcggagga aggtccgcga actggccgga gctgtccgaa gtgccaggca 300

cttggttgtc tacacgggcg ctggaatcag cacagcagct tctatcccag attatcgggg 360

tcctaatgga gtatggacac tgcttcagaa aggaaggcct gtgagtgctg ccgacctaag 420

cgaagcggag cctaccctca cccacatgag catcacccgt ttgcatgagc aaaagctggt 480

gcaacacgtg gtgtctcaga actgtgatgg gctccacctg cggagtgggt tgccccggac 540

cgccatctca gagctccatg ggaatatgta tattgaagtc tgcacctcct gcatccctaa 600

cagagagtat gtacgagtgt ttgatgtgac tgagcgtact gcccttcacc gacacctgac 660

aggccggacc tgccacaagt gcgggaccca gcttcgggat accattgtgc actttgggga 720

gagggggaca ttagggcagc ctctgaactg ggaggcagcg accgaggctg ctagcaaagc 780

agacacaatc ctgtgtttag ggtccagctt gaaggtacta aagaaatatc cccgcctctg 840

gtgcatgacg aagcctccaa gccgtcgacc caaactctac atcgtgaacc tgcagtggac 900

cccgaaggat gactgggctg ccctgaaact ccatgggaag tgtgatgatg tcatgcaact 960

cctcatgaat gaactgggcc tggagattcc tgtctacaac cggtggcagg atccaatctt 1020

ctccttggcg acccccctcc gtgctggtga agaaggcagc cacagtagga agtcactatg 1080

cagaagcaga gaagaagccc cacctgggga ccagagtgac ccccttgcct cagctccccc 1140

tatcctagga ggctggtttg gcaggggttg tgccaagcgt gcaaaaagga agaaagtggc 1200

atagcccgga gttgacaaag aacagttggc actttgcaga tggcttgacc cttgttcagg 1260

ggctccgccc gtgtcgccgt gaaggcgaga gtgccccctc cggctctagt gggtgccggc 1320

ttcgcggtga cggtcgtctt tagccatttg tccttgtgga aaggctctct tgcactgctg 1380

tgactgcccc gctatgggct tgatcttcaa ggacacctcc ctcctgttca cccctgtaca 1440

aaggtggcag caaggcctct gtgagaagag ctccggccct tcccaggcct ggccagcagg 1500

agagtccacc actgccttac ctcactctcc aggctagtct cagggcctcc ctctttctac 1560

tccttatcac gtacaagtgt tcactttata gaagcctttt tctgtattgg gtatgcggca 1620

cagggagaat ctaactaagc ctccagtctg agcaagtctt attaaacatc tctcaattgc 1680

taaaaaaaaa aaaaaaaaaa aaaaaaaaaa aa 1712

<210>  4
<211>  252
<212>  PRT
<213>  mus musculus

<400>  4

Met Ser Ile Thr Arg Leu His Glu Gln Lys Leu Val Gln His Val Val
1               5                   10                  15

Ser Gln Asn Cys Asp Gly Leu His Leu Arg Ser Gly Leu Pro Arg Thr
            20                  25                  30

Ala Ile Ser Glu Leu His Gly Asn Met Tyr Ile Glu Val Cys Thr Ser
        35                  40                  45

Cys Ile Pro Asn Arg Glu Tyr Val Arg Val Phe Asp Val Thr Glu Arg
    50                  55                  60

Thr Ala Leu His Arg His Leu Thr Gly Arg Thr Cys His Lys Cys Gly
65                  70                  75                  80

Thr Gln Leu Arg Asp Thr Ile Val His Phe Gly Glu Arg Gly Thr Leu
                85                  90                  95

Gly Gln Pro Leu Asn Trp Glu Ala Ala Thr Glu Ala Ala Ser Lys Ala
            100                 105                 110

Asp Thr Ile Leu Cys Leu Gly Ser Ser Leu Lys Val Leu Lys Lys Tyr
        115                 120                 125

Pro Arg Leu Trp Cys Met Thr Lys Pro Pro Ser Arg Arg Pro Lys Leu
    130                 135                 140

Tyr Ile Val Asn Leu Gln Trp Thr Pro Lys Asp Asp Trp Ala Ala Leu
145                 150                 155                 160

Lys Leu His Gly Lys Cys Asp Asp Val Met Gln Leu Leu Met Asn Glu
                165                 170                 175

Leu Gly Leu Glu Ile Pro Val Tyr Asn Arg Trp Gln Asp Pro Ile Phe
            180                 185                 190

Ser Leu Ala Thr Pro Leu Arg Ala Gly Glu Glu Gly Ser His Ser Arg
        195                 200                 205

Lys Ser Leu Cys Arg Ser Arg Glu Glu Ala Pro Pro Gly Asp Gln Ser
    210                 215                 220

Asp Pro Leu Ala Ser Ala Pro Pro Ile Leu Gly Gly Trp Phe Gly Arg
225                 230                 235                 240

Gly Cys Ala Lys Arg Ala Lys Arg Lys Lys Val Ala
                245                 250

<210>  5
<211>  1696
<212>  DNA
<213>  rattus norvegicus

<400>  5
atggcagccg gtggcggtct gagccgctcg gagcgcaaag cggctgagcg ggtccggagg      60

ttgcgggagg agcagcagcg ggagcgcctc cgtcaggtgt cacgcatcct gaggaaggcg     120

gctgcagagc gcagcgcgga ggagggccgg cttctagctg agagtgagga tctggtgacc     180

gagcttcagg gtcggagccg gcggcgtgag ggcctcaagc gccgccagga ggaggtgtgt     240

gatgacccag aggagttgcg gaggaaggtc cgcgaactag ccggagctgt ccgaagtgcc     300

agacacttgg ttgtctacac aggcgctggg atcagcacgg cagcctctat cccagattac     360

cggggtccta atggagtgtg gacactgctc cagaaaggaa ggcctgtgag tgctgccgac     420

ctaagcgaag cagagcctac cctcacccac atgagcatca cccagctgca caagcataag     480

ctggtgcaac acgtggtgtc tcagaactgc gacgggctcc acctgcgcag tgggctgcca     540

cggaccgcca tctctgagct ccatgggaat atgtatattg aagtctgcac ctcctgcatc     600

cctaacagag agtacgtccg agtgtttgac gtgacggagc gtaccgccct tcaccgacac     660

ctgacaggcc ggacgtgcca caagtgtggg actcagcttc gggacaccat tgtgcacttt     720

ggggagaggg ggacgctagg gcagcccctg aactgggagg cagcgactga ggccgctagc     780

aaagcagaca caatcctgtg tttagggtcc agcttgaagg tactgaagaa gtacccccgt     840

ctctggtgca tgacgaagcc cccaagccgt cggcccaaac tctacattgt gaacctgcag     900

tggaccccga aggatgactg ggctgccctg aagcttcatg ggaaatgtga tgatgtaatg     960

cggctcctca tggacgaact gggcctggag atccctgtct acaaccggtg gcaggaccca    1020

atcttctcct tggcaactcc actccgtgct ggcgaagaag gtagccacag taggaagtca    1080

ctgtgcagga gcagagaaga gcccccaccc ggggaccaga gtgcccctct tgcctcagcc    1140

acccccatcc taggaggctg gttcggcagg ggctgtgcta agcgtgcaaa aaggaagaaa    1200

gcggcgtagc cccggtgttg acaaagagca gtcggcacct tgcagatggc ttgacccatg    1260

tttagggtcc tgtgtcacag tgaaggctag agtgccccca ccagatctag tggaaagaac    1320

tctggggttg ggggtgacac ttcctggtga cggtcgtttt tagccatctg tccttgtgga    1380

aagcctcact tgcactgctg tgatcgcccc actacgggct tgatcttcaa ggacacctcc    1440

atcctatccc cccctataca aaggtggcag caaggccttt gcaagaagag ctcccgtctt    1500

tcccaggcct ggcccacaga ggagtccacc actgccttac ctcactctcc aggctagtct 1560

cagggcctcc ctctttctac tacttactac ttaaaagtgt tcactttata gaaacctttt 1620

tctgtattgg gtatgcggca cagggaggat ctaagcctcc ggtctgagca agtcttatta 1680

aacatctctc tattgc 1696

<210> 6
<211> 402
<212> PRT
<213> rattus norvegicus

<400> 6

Met Ala Ala Gly Gly Gly Leu Ser Arg Ser Glu Arg Lys Ala Ala Glu
1               5                   10                  15

Arg Val Arg Arg Leu Arg Glu Glu Gln Gln Arg Glu Arg Leu Arg Gln
            20                  25                  30

Val Ser Arg Ile Leu Arg Lys Ala Ala Ala Glu Arg Ser Ala Glu Glu
        35                  40                  45

Gly Arg Leu Leu Ala Glu Ser Glu Asp Leu Val Thr Glu Leu Gln Gly
    50                  55                  60

Arg Ser Arg Arg Arg Glu Gly Leu Lys Arg Arg Gln Glu Glu Val Cys
65                  70                  75                  80

Asp Asp Pro Glu Glu Leu Arg Arg Lys Val Arg Glu Leu Ala Gly Ala
                85                  90                  95

Val Arg Ser Ala Arg His Leu Val Val Tyr Thr Gly Ala Gly Ile Ser
            100                 105                 110

Thr Ala Ala Ser Ile Pro Asp Tyr Arg Gly Pro Asn Gly Val Trp Thr
        115                 120                 125

Leu Leu Gln Lys Gly Arg Pro Val Ser Ala Ala Asp Leu Ser Glu Ala
    130                 135                 140

Glu Pro Thr Leu Thr His Met Ser Ile Thr Gln Leu His Lys His Lys
145                 150                 155                 160

Leu Val Gln His Val Val Ser Gln Asn Cys Asp Gly Leu His Leu Arg
                165                 170                 175

Ser Gly Leu Pro Arg Thr Ala Ile Ser Glu Leu His Gly Asn Met Tyr
            180                 185                 190

Ile Glu Val Cys Thr Ser Cys Ile Pro Asn Arg Glu Tyr Val Arg Val
        195                 200                 205

Phe Asp Val Thr Glu Arg Thr Ala Leu His Arg His Leu Thr Gly Arg
    210                 215                 220

Thr Cys His Lys Cys Gly Thr Gln Leu Arg Asp Thr Ile Val His Phe
225                 230                 235                 240

Gly Glu Arg Gly Thr Leu Gly Gln Pro Leu Asn Trp Glu Ala Ala Thr
                245                 250                 255

Glu Ala Ala Ser Lys Ala Asp Thr Ile Leu Cys Leu Gly Ser Ser Leu
            260                 265                 270

Lys Val Leu Lys Lys Tyr Pro Arg Leu Trp Cys Met Thr Lys Pro Pro
        275                 280                 285

Ser Arg Arg Pro Lys Leu Tyr Ile Val Asn Leu Gln Trp Thr Pro Lys
    290                 295                 300

Asp Asp Trp Ala Ala Leu Lys Leu His Gly Lys Cys Asp Asp Val Met
305                 310                 315                 320

Arg Leu Leu Met Asp Glu Leu Gly Leu Glu Ile Pro Val Tyr Asn Arg
                325                 330                 335

Trp Gln Asp Pro Ile Phe Ser Leu Ala Thr Pro Leu Arg Ala Gly Glu
            340                 345                 350

Glu Gly Ser His Ser Arg Lys Ser Leu Cys Arg Ser Arg Glu Glu Pro
        355                 360                 365

Pro Pro Gly Asp Gln Ser Ala Pro Leu Ala Ser Ala Thr Pro Ile Leu
    370                 375                 380

Gly Gly Trp Phe Gly Arg Gly Cys Ala Lys Arg Ala Lys Arg Lys Lys
385                 390                 395                 400

Ala Ala

<210>  7
<211>  1718
<212>  DNA
<213>  homo sapiens

<400>  7
gcggaagcgg aagagcaggt ctccagggga gcgatggcag ccgggggtct gagccgctcc      60

gagcgcaaag cggcggagcg ggtccggagg ttgcgggagg agcagcagag ggagcgcctc 120
cgccaggtgt cgcgcatcct gaggaaggcg gcggcggagc gcagcgccga ggagggccgg 180
ctgctggccg agagcgcgga cctggtaacg gagctgcagg gccggagccg gcggcgcgag 240
ggcctgaagc ggcggcagga ggaggtgtgc gacgacccgg aggagctgcg ggggaaggtc 300
cgggagctgg ccagcgccgt ccggaacgcc aaatacttgg tcgtctacac aggcgcggga 360
atcagcacgg cagcgtctat cccagactac cggggcccta atggagtgtg gacactgctt 420
cagaaaggga gaagcgttag tgctgccgac ctgagcgagg ccgagccaac cctcacccac 480
atgagcatca cccgtctgca tgagcagaag ctggtgcagc atgtggtgtc tcagaactgt 540
gacgggctcc acctgaggag tgggctgccg cgcacggcca tctccgagct ccacgggaac 600
atgtacattg aagtctgtac ctcctgcgtt cccaacaggg agtacgtgcg ggtgttcgat 660
gtgacggagc gcactgccct ccacagacac cagacaggcc ggacctgcca caagtgtggg 720
acccagctgc gggacaccat tgtgcacttt ggggagaggg ggacgttggg gcagcctctg 780
aactgggaag cggcgaccga ggctgccagc agagcagaca ccatcctgtg tctagggtcc 840
agcctgaagg ttctaaagaa gtacccacgc ctctggtgca tgaccaagcc ccctagccgg 900
cggccgaagc tttacatcgt gaacctgcag tggaccccga aggatgactg ggctgccctg 960
aagctacatg ggaagtgtga tgacgtcatg cggctcctca tggccgagct gggcttggag 1020
atccccgcct atagcaggtg gcaggatccc attttctcac tggcgactcc cctgcgtgct 1080
ggtgaagaag gcagccacag tcggaagtcg ctgtgcagaa gcagagagga ggccccgcct 1140
ggggaccggg gtgcaccgct tagctcggcc cccatcctag ggggctggtt tggcaggggc 1200
tgcacaaaac gcacaaaaag gaagaaagtg acgtaatcac gtgctcgatg aagaacagtt 1260
ggcactttgc agatggccag tgtcacggtg aaggctgggt tgcccccacg ggtctaggga 1320
gaacgaactc tttggggatg acattttcac cgtgacattt ttagccattt gtccttgagg 1380
aagccccttg cactgctgcg gttgtaccct gatacggcct ggccatcgag gacacctgcc 1440
catccggcct ctgtgtcaag aggtggcagc cgcacctttc tgtgagaacg gaactcgggt 1500
tatttcagcc ccggcctgca gagtggaagc gcccagcggc ctttcctcgc tcaccaggcc 1560
agtctcaggg cctcaccgta tttctactac tacttaatga aaaagtgtga actttataga 1620
atcctctctg tactggatgt gcggcagagg ggtggctccg agcctcggct ctatgcagac 1680
ctttttattt ctattaaacg tttctgcact ggcaaaaa 1718

<210> 8
<211> 400
<212> PRT
<213> homo sapiens

<400> 8

Met Ala Ala Gly Gly Leu Ser Arg Ser Glu Arg Lys Ala Ala Glu Arg
1               5                   10                  15

Val Arg Arg Leu Arg Glu Glu Gln Gln Arg Glu Arg Leu Arg Gln Val
            20                  25                  30

Ser Arg Ile Leu Arg Lys Ala Ala Ala Glu Arg Ser Ala Glu Glu Gly
        35                  40                  45

Arg Leu Leu Ala Glu Ser Ala Asp Leu Val Thr Glu Leu Gln Gly Arg
    50                  55                  60

Ser Arg Arg Arg Glu Gly Leu Lys Arg Arg Gln Glu Glu Val Cys Asp
65                  70                  75                  80

Asp Pro Glu Glu Leu Arg Gly Lys Val Arg Glu Leu Ala Ser Ala Val
                85                  90                  95

Arg Asn Ala Lys Tyr Leu Val Val Tyr Thr Gly Ala Gly Ile Ser Thr
            100                 105                 110

Ala Ala Ser Ile Pro Asp Tyr Arg Gly Pro Asn Gly Val Trp Thr Leu
        115                 120                 125

Leu Gln Lys Gly Arg Ser Val Ser Ala Ala Asp Leu Ser Glu Ala Glu
    130                 135                 140

Pro Thr Leu Thr His Met Ser Ile Thr Arg Leu His Glu Gln Lys Leu
145                 150                 155                 160

Val Gln His Val Val Ser Gln Asn Cys Asp Gly Leu His Leu Arg Ser
                165                 170                 175

Gly Leu Pro Arg Thr Ala Ile Ser Glu Leu His Gly Asn Met Tyr Ile
            180                 185                 190

Glu Val Cys Thr Ser Cys Val Pro Asn Arg Glu Tyr Val Arg Val Phe
        195                 200                 205

Asp Val Thr Glu Arg Thr Ala Leu His Arg His Gln Thr Gly Arg Thr
    210                 215                 220

Cys His Lys Cys Gly Thr Gln Leu Arg Asp Thr Ile Val His Phe Gly
225                 230                 235                 240

Glu Arg Gly Thr Leu Gly Gln Pro Leu Asn Trp Glu Ala Ala Thr Glu
                245                 250                 255

Ala Ala Ser Arg Ala Asp Thr Ile Leu Cys Leu Gly Ser Ser Leu Lys
            260                 265                 270

Val Leu Lys Lys Tyr Pro Arg Leu Trp Cys Met Thr Lys Pro Pro Ser
        275                 280                 285

Arg Arg Pro Lys Leu Tyr Ile Val Asn Leu Gln Trp Thr Pro Lys Asp
    290                 295                 300

Asp Trp Ala Ala Leu Lys Leu His Gly Lys Cys Asp Asp Val Met Arg
305                 310                 315                 320

Leu Leu Met Ala Glu Leu Gly Leu Glu Ile Pro Ala Tyr Ser Arg Trp
                325                 330                 335

Gln Asp Pro Ile Phe Ser Leu Ala Thr Pro Leu Arg Ala Gly Glu Glu
            340                 345                 350

Gly Ser His Ser Arg Lys Ser Leu Cys Arg Ser Arg Glu Glu Ala Pro
        355                 360                 365

Pro Gly Asp Arg Gly Ala Pro Leu Ser Ser Ala Pro Ile Leu Gly Gly
    370                 375                 380

Trp Phe Gly Arg Gly Cys Thr Lys Arg Thr Lys Arg Lys Lys Val Thr
385                 390                 395                 400

<210>  9
<211>  17
<212>  DNA
<213>  artificial sequence

<220>
<223>  primer for ACC1

<400>  9
caggccggcc aggtttg                                                    17

<210>  10
<211>  20
<212>  DNA
<213>  artificial sequence

<220>
<223>  primer for ACC1

<400>  10
tccatgtgcc gagggttgat                                                 20

<210>  11
<211>  24
<212>  DNA
<213>  artificial sequence

<220>
<223>  primer for ACLY

<400>  11
gaggtggccc caactatcaa gagg                                              24


<210>  12
<211>  24
<212>  DNA
<213>  artificial sequence

<220>
<223>  primer for ACLY

<400>  12
cccgctggca ttaaggagga agtt                                              24


<210>  13
<211>  24
<212>  DNA
<213>  artificial sequence

<220>
<223>  primer for PGC-1a

<400>  13
tacaatgaat gcagcggtct tagc                                              24


<210>  14
<211>  22
<212>  DNA
<213>  artificial sequence

<220>
<223>  primer for PGC-1a

<400>  14
gaggagggtc atcgtttgtg gt                                                22


<210>  15
<211>  24
<212>  DNA
<213>  artificial sequence

<220>
<223>  primer for PGC-1b

<400>  15
gccctggaaa gcccctgtga gagt                                              24


<210>  16
<211>  24
<212>  DNA
<213>  artificial sequence

<220>
<223>  primer for PGC-1b

<400>  16
gtgtggtggg tggcgtgagt cctg                                              24


<210>  17
<211>  19
<212>  DNA
<213>  artificial sequence

<220>
<223>  primer for sirt7

<400>  17
ccccggaccg ccatctcag                                                    19


<210>  18
<211>  23
<212>  DNA
<213>  artificial sequence

<220>
<223>  primer for sirt7

<400>  18
atctccaggc ccagttcatt cat                                               23

SEQUENCE LISTING

<110>  Max-Planck-Gesellschaft zur Förderung der Wissenschaften
       e.V.

<120>  Pharmaceutical compositions for treating age-related diseases and
        methods for screening compounds suitable for treating
       age-related diseases

<130>  M 2522 EP

<160>  18

<170>  PatentIn version 3.3

<210>  1
<211>  1711
<212>  DNA
<213>  mouse

<400>  1
gcagccggtg gcggtctgag ccgctcggag cgcaaagctg ctgagcgggt ccggaggctg      60

cgggaggagc agcagcggga gcgcctccgc caggtgtcac gcatcctgag gaaggcggct     120

gcagagcgca gcgcggagga gggccggctc ttggccgaga gcgaggatct ggtgaccgag     180

ctgcagggtc gaagtcggcg gcgtgagggc ctcaagcgcc gccaggagga ggtgtgtgat     240

gacccggagg agctgcggag gaaggtccgc gaactggccg gagctgtccg aagtgccagg     300

cacttggttg tctacacggg cgctggaatc agcacagcag cttctatccc agattatcgg     360

ggtcctaatg gagtatggac actgcttcag aaaggaaggc ctgtgagtgc tgccgaccta     420

agcgaagcgg agcctaccct cacccacatg agcatcaccc gtttgcatga gcaaaagctg     480

gtgcaacacg tggtgtctca gaactgtgat gggctccacc tgcggagtgg gttgccccgg     540

accgccatct cagagctcca tgggaatatg tatattgaag tctgcacctc ctgcatccct     600

aacagagagt atgtacgagt gtttgatgtg actgagcgta ctgcccttca ccgacacctg     660

acaggccgga cctgccacaa gtgcgggacc cagcttcggg ataccattgt gcactttggg     720

gagaggggga cattagggca gcctctgaac tgggaggcag cgaccgaggc tgctagcaaa     780

gcagacacaa tcctgtgttt agggtccagc ttgaaggtac taaagaaata tccccgcctc     840

tggtgcatga cgaagcctcc aagccgtcga cccaaactct acatcgtgaa cctgcagtgg     900

accccgaagg atgactgggc tgccctgaaa ctccatggga agtgtgatga tgtcatgcaa     960

ctcctcatga atgaactggg cctggagatt cctgtctaca accggtggca ggatccaatc    1020

ttctccttgg cgacccccct ccgtgctggt gaagaaggca gccacagtag gaagtcacta    1080

tgcagaagca gagaagaagc cccacctggg gaccagagtg acccccttgc ctcagctccc    1140

cctatcctag gaggctggtt tggcaggggt tgtgccaagc gtgcaaaaag gaagaaagtg    1200

gcatagcccg gagttgacaa agaacagttg gcactttgca tatggcttga cccttgttca    1260

ggggctccgc ccgtgtcccg taaggcgaga gtgccccctc cggctctagt gggtgccggc    1320

ttcgcggtga cggtcgtctt tagccatttg tccttgtgga aaggctctct tgcactgctg    1380

tgactgcccc gctatgggct tgatcttcaa ggacacctcc ctcctgttca cccctgtaca    1440

aaggtggcag caaggcctct gtgagaaagc tccggccctt cccaggcctg gccagcagga 1500

gagtccacca ctgccttacc tcactctcca ggctagtctc agggcctccc tctttctact 1560

ccttatcacg tacaagtgtt cactttatag aagccttttt ctgtattggg tatgcggcac 1620

agggagaatc taactaagcc tccagtctga gcaagtctta ttaaacatct ctcaattgca 1680

aaaaaaaaaa aaaaaaaaaa aaaaaaaaaa a 1711

<210> 2
<211> 402
<212> PRT
<213> mouse

<400> 2

Met Ala Ala Gly Gly Gly Leu Ser Arg Ser Glu Arg Lys Ala Ala Glu
1               5                   10                  15

Arg Val Arg Arg Leu Arg Glu Glu Gln Gln Arg Glu Arg Leu Arg Gln
            20                  25                  30

Val Ser Arg Ile Leu Arg Lys Ala Ala Ala Glu Arg Ser Ala Glu Glu
        35                  40                  45

Gly Arg Leu Leu Ala Glu Ser Glu Asp Leu Val Thr Glu Leu Gln Gly
    50                  55                  60

Arg Ser Arg Arg Arg Glu Gly Leu Lys Arg Arg Gln Glu Glu Val Cys
65                  70                  75                  80

Asp Asp Pro Glu Glu Leu Arg Arg Lys Val Arg Glu Leu Ala Gly Ala
                85                  90                  95

Val Arg Ser Ala Arg His Leu Val Val Tyr Thr Gly Ala Gly Ile Ser
            100                 105                 110

Thr Ala Ala Ser Ile Pro Asp Tyr Arg Gly Pro Asn Gly Val Trp Thr
        115                 120                 125

Leu Leu Gln Lys Gly Arg Pro Val Ser Ala Ala Asp Leu Ser Glu Ala
    130                 135                 140

Glu Pro Thr Leu Thr His Met Ser Ile Thr Arg Leu His Glu Gln Lys
145                 150                 155                 160

Leu Val Gln His Val Val Ser Gln Asn Cys Asp Gly Leu His Leu Arg
                165                 170                 175

Ser Gly Leu Pro Arg Thr Ala Ile Ser Glu Leu His Gly Asn Met Tyr
            180                 185                 190

Ile Glu Val Cys Thr Ser Cys Ile Pro Asn Arg Glu Tyr Val Arg Val

195                    200                    205

Phe Asp Val Thr Glu Arg Thr Ala Leu His Arg His Leu Thr Gly Arg
    210                    215                    220

Thr Cys His Lys Cys Gly Thr Gln Leu Arg Asp Thr Ile Val His Phe
225                    230                    235                    240

Gly Glu Arg Gly Thr Leu Gly Gln Pro Leu Asn Trp Glu Ala Ala Thr
                245                    250                    255

Glu Ala Ala Ser Lys Ala Asp Thr Ile Leu Cys Leu Gly Ser Ser Leu
            260                    265                    270

Lys Val Leu Lys Lys Tyr Pro Arg Leu Trp Cys Met Thr Lys Pro Pro
        275                    280                    285

Ser Arg Arg Pro Lys Leu Tyr Ile Val Asn Leu Gln Trp Thr Pro Lys
    290                    295                    300

Asp Asp Trp Ala Ala Leu Lys Leu His Gly Lys Cys Asp Asp Val Met
305                    310                    315                    320

Gln Leu Leu Met Asn Glu Leu Gly Leu Glu Ile Pro Val Tyr Asn Arg
                325                    330                    335

Trp Gln Asp Pro Ile Phe Ser Leu Ala Thr Pro Leu Arg Ala Gly Glu
            340                    345                    350

Glu Gly Ser His Ser Arg Lys Ser Leu Cys Arg Ser Arg Glu Glu Ala
        355                    360                    365

Pro Pro Gly Asp Gln Ser Asp Pro Leu Ala Ser Ala Pro Pro Ile Leu
    370                    375                    380

Gly Gly Trp Phe Gly Arg Gly Cys Ala Lys Arg Ala Lys Arg Lys Lys
385                    390                    395                    400

Val Ala

<210>  3
<211>  1712
<212>  DNA
<213>  mouse

<400>  3
agccggtggc ggtctgagcc gctcggagcg caaagctgct gagcgggtcc ggaggctgcg      60

ggaggagcag cagcgggagc gcctccgcca ggtgtcacgc atcctgagga aggcggctgc     120

agagcgcagc gcggaggagg gccggctctt ggccgagagc gaggatctgg tgaccgagct     180

gcagggtcga agtcggcggc gtgagggcct caagcgccgc caggaggagg tgtgtgatga     240

cccggaggag ctgcggagga aggtccgcga actggccgga gctgtccgaa gtgccaggca 300
cttggttgtc tacacgggcg ctggaatcag cacagcagct tctatcccag attatcgggg 360
tcctaatgga gtatggacac tgcttcagaa aggaaggcct gtgagtgctg ccgacctaag 420
cgaagcggag cctaccctca cccacatgag catcacccgt ttgcatgagc aaaagctggt 480
gcaacacgtg gtgtctcaga actgtgatgg gctccacctg cggagtgggt tgccccggac 540
cgccatctca gagctccatg ggaatatgta tattgaagtc tgcacctcct gcatccctaa 600
cagagagtat gtacgagtgt ttgatgtgac tgagcgtact gcccttcacc gacacctgac 660
aggccggacc tgccacaagt gcgggaccca gcttcgggat accattgtgc actttgggga 720
gagggggaca ttagggcagc ctctgaactg ggaggcagcg accgaggctg ctagcaaagc 780
agacacaatc ctgtgtttag ggtccagctt gaaggtacta aagaaatatc cccgcctctg 840
gtgcatgacg aagcctccaa gccgtcgacc caaactctac atcgtgaacc tgcagtggac 900
cccgaaggat gactgggctg ccctgaaact ccatgggaag tgtgatgatg tcatgcaact 960
cctcatgaat gaactgggcc tggagattcc tgtctacaac cggtggcagg atccaatctt 1020
ctccttggcg acccccctcc gtgctggtga agaaggcagc cacagtagga agtcactatg 1080
cagaagcaga gaagaagccc cacctgggga ccagagtgac ccccttgcct cagctccccc 1140
tatcctagga ggctggtttg gcaggggttg tgccaagcgt gcaaaaagga agaaagtggc 1200
atagcccgga gttgacaaag aacagttggc actttgcaga tggcttgacc cttgttcagg 1260
ggctccgccc gtgtcgccgt gaaggcgaga gtgccccctc cggctctagt gggtgccggc 1320
ttcgcggtga cggtcgtctt tagccatttg tccttgtgga aaggctctct tgcactgctg 1380
tgactgcccc gctatgggct tgatcttcaa ggacacctcc ctcctgttca cccctgtaca 1440
aaggtggcag caaggcctct gtgagaagag ctccggccct tcccaggcct ggccagcagg 1500
agagtccacc actgccttac ctcactctcc aggctagtct cagggcctcc ctctttctac 1560
tccttatcac gtacaagtgt tcactttata gaagcctttt tctgtattgg gtatgcggca 1620
cagggagaat ctaactaagc ctccagtctg agcaagtctt attaaacatc tctcaattgc 1680
taaaaaaaaa aaaaaaaaaa aaaaaaaaaa aa 1712

<210> 4
<211> 252
<212> PRT
<213> mouse

<400> 4

Met Ser Ile Thr Arg Leu His Glu Gln Lys Leu Val Gln His Val Val
1               5                   10                  15

Ser Gln Asn Cys Asp Gly Leu His Leu Arg Ser Gly Leu Pro Arg Thr
            20                  25                  30

Ala Ile Ser Glu Leu His Gly Asn Met Tyr Ile Glu Val Cys Thr Ser

35                  40                  45

Cys Ile Pro Asn Arg Glu Tyr Val Arg Val Phe Asp Val Thr Glu Arg
    50                  55                  60

Thr Ala Leu His Arg His Leu Thr Gly Arg Thr Cys His Lys Cys Gly
65                  70                  75                  80

Thr Gln Leu Arg Asp Thr Ile Val His Phe Gly Glu Arg Gly Thr Leu
                85                  90                  95

Gly Gln Pro Leu Asn Trp Glu Ala Ala Thr Glu Ala Ala Ser Lys Ala
            100                 105                 110

Asp Thr Ile Leu Cys Leu Gly Ser Ser Leu Lys Val Leu Lys Lys Tyr
        115                 120                 125

Pro Arg Leu Trp Cys Met Thr Lys Pro Pro Ser Arg Arg Pro Lys Leu
    130                 135                 140

Tyr Ile Val Asn Leu Gln Trp Thr Pro Lys Asp Asp Trp Ala Ala Leu
145                 150                 155                 160

Lys Leu His Gly Lys Cys Asp Asp Val Met Gln Leu Leu Met Asn Glu
                165                 170                 175

Leu Gly Leu Glu Ile Pro Val Tyr Asn Arg Trp Gln Asp Pro Ile Phe
            180                 185                 190

Ser Leu Ala Thr Pro Leu Arg Ala Gly Glu Glu Gly Ser His Ser Arg
        195                 200                 205

Lys Ser Leu Cys Arg Ser Arg Glu Glu Ala Pro Pro Gly Asp Gln Ser
    210                 215                 220

Asp Pro Leu Ala Ser Ala Pro Pro Ile Leu Gly Gly Trp Phe Gly Arg
225                 230                 235                 240

Gly Cys Ala Lys Arg Ala Lys Arg Lys Lys Val Ala
                245                 250

<210>  5
<211>  1696
<212>  DNA
<213>  rat

<400>  5
atggcagccg gtggcggtct gagccgctcg gagcgcaaag cggctgagcg ggtccggagg      60

ttgcgggagg agcagcagcg ggagcgcctc cgtcaggtgt cacgcatcct gaggaaggcg     120

gctgcagagc gcagcgcgga ggagggccgg cttctagctg agagtgagga tctggtgacc     180

gagcttcagg gtcggagccg gcggcgtgag ggcctcaagc gccgccagga ggaggtgtgt     240

gatgacccag aggagttgcg gaggaaggtc cgcgaactag ccggagctgt ccgaagtgcc 300
agacacttgg ttgtctacac aggcgctggg atcagcacgg cagcctctat cccagattac 360
cggggtccta atggagtgtg gacactgctc cagaaaggaa ggcctgtgag tgctgccgac 420
ctaagcgaag cagagcctac cctcacccac atgagcatca cccagctgca caagcataag 480
ctggtgcaac acgtggtgtc tcagaactgc gacgggctcc acctgcgcag tgggctgcca 540
cggaccgcca tctctgagct ccatgggaat atgtatattg aagtctgcac ctcctgcatc 600
cctaacagag agtacgtccg agtgtttgac gtgacggagc gtaccgccct tcaccgacac 660
ctgacaggcc ggacgtgcca caagtgtggg actcagcttc gggacaccat tgtgcacttt 720
ggggagaggg ggacgctagg gcagcccctg aactgggagg cagcgactga ggccgctagc 780
aaagcagaca caatcctgtg tttagggtcc agcttgaagg tactgaagaa gtacccccgt 840
ctctggtgca tgacgaagcc cccaagccgt cggcccaaac tctacattgt gaacctgcag 900
tggaccccga aggatgactg ggctgccctg aagcttcatg ggaaatgtga tgatgtaatg 960
cggctcctca tggacgaact gggcctggag atccctgtct acaaccggtg gcaggaccca 1020
atcttctcct tggcaactcc actccgtgct ggcgaagaag gtagccacag taggaagtca 1080
ctgtgcagga gcagagaaga gcccccaccc ggggaccaga gtgcccctct tgcctcagcc 1140
acccccatcc taggaggctg gttcggcagg ggctgtgcta agcgtgcaaa aaggaagaaa 1200
gcggcgtagc cccggtgttg acaaagagca gtcggcacct tgcagatggc ttgacccatg 1260
tttagggtcc tgtgtcacag tgaaggctag agtgccccca ccagatctag tggaaagaac 1320
tctggggttg ggggtgacac ttcctggtga cggtcgtttt tagccatctg tccttgtgga 1380
aagcctcact tgcactgctg tgatcgcccc actacgggct tgatcttcaa ggacacctcc 1440
atcctatccc cccctataca aaggtggcag caaggccttt gcaagaagag ctcccgtctt 1500
tcccaggcct ggcccacaga ggagtccacc actgccttac ctcactctcc aggctagtct 1560
cagggcctcc ctctttctac tacttactac ttaaaagtgt tcactttata gaaacctttt 1620
tctgtattgg gtatgcggca cagggaggat ctaagcctcc ggtctgagca agtcttatta 1680
aacatctctc tattgc 1696

<210> 6
<211> 402
<212> PRT
<213> rat

<400> 6

Met Ala Ala Gly Gly Gly Leu Ser Arg Ser Glu Arg Lys Ala Ala Glu
1               5                   10                  15

Arg Val Arg Arg Leu Arg Glu Glu Gln Gln Arg Glu Arg Leu Arg Gln
            20                  25                  30

Val Ser Arg Ile Leu Arg Lys Ala Ala Ala Glu Arg Ser Ala Glu Glu

        35                  40                  45

Gly Arg Leu Leu Ala Glu Ser Glu Asp Leu Val Thr Glu Leu Gln Gly
    50                  55                  60

Arg Ser Arg Arg Arg Glu Gly Leu Lys Arg Arg Gln Glu Glu Val Cys
65                  70                  75                  80

Asp Asp Pro Glu Glu Leu Arg Arg Lys Val Arg Glu Leu Ala Gly Ala
                85                  90                  95

Val Arg Ser Ala Arg His Leu Val Val Tyr Thr Gly Ala Gly Ile Ser
            100                 105                 110

Thr Ala Ala Ser Ile Pro Asp Tyr Arg Gly Pro Asn Gly Val Trp Thr
        115                 120                 125

Leu Leu Gln Lys Gly Arg Pro Val Ser Ala Ala Asp Leu Ser Glu Ala
    130                 135                 140

Glu Pro Thr Leu Thr His Met Ser Ile Thr Gln Leu His Lys His Lys
145                 150                 155                 160

Leu Val Gln His Val Val Ser Gln Asn Cys Asp Gly Leu His Leu Arg
                165                 170                 175

Ser Gly Leu Pro Arg Thr Ala Ile Ser Glu Leu His Gly Asn Met Tyr
            180                 185                 190

Ile Glu Val Cys Thr Ser Cys Ile Pro Asn Arg Glu Tyr Val Arg Val
        195                 200                 205

Phe Asp Val Thr Glu Arg Thr Ala Leu His Arg His Leu Thr Gly Arg
    210                 215                 220

Thr Cys His Lys Cys Gly Thr Gln Leu Arg Asp Thr Ile Val His Phe
225                 230                 235                 240

Gly Glu Arg Gly Thr Leu Gly Gln Pro Leu Asn Trp Glu Ala Ala Thr
                245                 250                 255

Glu Ala Ala Ser Lys Ala Asp Thr Ile Leu Cys Leu Gly Ser Ser Leu
            260                 265                 270

Lys Val Leu Lys Lys Tyr Pro Arg Leu Trp Cys Met Thr Lys Pro Pro
        275                 280                 285

Ser Arg Arg Pro Lys Leu Tyr Ile Val Asn Leu Gln Trp Thr Pro Lys
    290                 295                 300

Asp Asp Trp Ala Ala Leu Lys Leu His Gly Lys Cys Asp Asp Val Met

305                 310                 315                 320

Arg Leu Leu Met Asp Glu Leu Gly Leu Glu Ile Pro Val Tyr Asn Arg
                325                 330                 335

Trp Gln Asp Pro Ile Phe Ser Leu Ala Thr Pro Leu Arg Ala Gly Glu
            340                 345                 350

Glu Gly Ser His Ser Arg Lys Ser Leu Cys Arg Ser Arg Glu Glu Pro
        355                 360                 365

Pro Pro Gly Asp Gln Ser Ala Pro Leu Ala Ser Ala Thr Pro Ile Leu
    370                 375                 380

Gly Gly Trp Phe Gly Arg Gly Cys Ala Lys Arg Ala Lys Arg Lys Lys
385                 390                 395                 400

Ala Ala

<210>  7
<211>  1718
<212>  DNA
<213>  human

<400>  7
gcggaagcgg aagagcaggt ctccagggga gcgatggcag ccgggggtct gagccgctcc      60

gagcgcaaag cggcggagcg ggtccggagg ttgcgggagg agcagcagag ggagcgcctc     120

cgccaggtgt cgcgcatcct gaggaaggcg gcggcggagc gcagcgccga ggagggccgg     180

ctgctggccg agagcgcgga cctggtaacg gagctgcagg gccggagccg gcggcgcgag     240

ggcctgaagc ggcggcagga ggaggtgtgc gacgacccgg aggagctgcg ggggaaggtc     300

cgggagctgg ccagcgccgt ccggaacgcc aaatacttgg tcgtctacac aggcgcggga     360

atcagcacgg cagcgtctat cccagactac cggggcccta atggagtgtg gacactgctt     420

cagaaaggga gaagcgttag tgctgccgac ctgagcgagg ccgagccaac cctcacccac     480

atgagcatca cccgtctgca tgagcagaag ctggtgcagc atgtggtgtc tcagaactgt     540

gacgggctcc acctgaggag tgggctgccg cgcacggcca tctccgagct ccacgggaac     600

atgtacattg aagtctgtac ctcctgcgtt cccaacaggg agtacgtgcg ggtgttcgat     660

gtgacggagc gcactgccct ccacagacac cagacaggcc ggacctgcca caagtgtggg     720

acccagctgc gggacaccat tgtgcacttt ggggagaggg ggacgttggg gcagcctctg     780

aactgggaag cggcgaccga ggctgccagc agagcagaca ccatcctgtg tctagggtcc     840

agcctgaagg ttctaaagaa gtacccacgc ctctggtgca tgaccaagcc ccctagccgg     900

cggccgaagc tttacatcgt gaacctgcag tggaccccga aggatgactg ggctgccctg     960

aagctacatg ggaagtgtga tgacgtcatg cggctcctca tggccgagct gggcttggag    1020

atccccgcct atagcaggtg gcaggatccc attttctcac tggcgactcc cctgcgtgct    1080

ggtgaagaag gcagccacag tcggaagtcg ctgtgcagaa gcagagagga ggccccgcct 1140
ggggaccggg gtgcaccgct tagctcggcc cccatcctag ggggctggtt tggcaggggc 1200
tgcacaaaac gcacaaaaag gaagaaagtg acgtaatcac gtgctcgatg aagaacagtt 1260
ggcactttgc agatggccag tgtcacggtg aaggctgggt tgcccccacg ggtctaggga 1320
gaacgaactc tttggggatg acattttcac cgtgacattt ttagccattt gtccttgagg 1380
aagccccttg cactgctgcg gttgtaccct gatacggcct ggccatcgag gacacctgcc 1440
catccggcct ctgtgtcaag aggtggcagc cgcacctttc tgtgagaacg gaactcgggt 1500
tatttcagcc ccggcctgca gagtggaagc gcccagcggc ctttcctcgc tcaccaggcc 1560
agtctcaggg cctcaccgta tttctactac tacttaatga aaaagtgtga actttataga 1620
atcctctctg tactggatgt gcggcagagg ggtggctccg agcctcggct ctatgcagac 1680
ctttttattt ctattaaacg tttctgcact ggcaaaaa 1718

<210> 8
<211> 400
<212> PRT
<213> human

<400> 8

Met Ala Ala Gly Gly Leu Ser Arg Ser Glu Arg Lys Ala Ala Glu Arg
1               5                   10                  15

Val Arg Arg Leu Arg Glu Glu Gln Gln Arg Glu Arg Leu Arg Gln Val
            20                  25                  30

Ser Arg Ile Leu Arg Lys Ala Ala Ala Glu Arg Ser Ala Glu Glu Gly
        35                  40                  45

Arg Leu Leu Ala Glu Ser Ala Asp Leu Val Thr Glu Leu Gln Gly Arg
    50                  55                  60

Ser Arg Arg Arg Glu Gly Leu Lys Arg Arg Gln Glu Glu Val Cys Asp
65                  70                  75                  80

Asp Pro Glu Glu Leu Arg Gly Lys Val Arg Glu Leu Ala Ser Ala Val
                85                  90                  95

Arg Asn Ala Lys Tyr Leu Val Val Tyr Thr Gly Ala Gly Ile Ser Thr
            100                 105                 110

Ala Ala Ser Ile Pro Asp Tyr Arg Gly Pro Asn Gly Val Trp Thr Leu
        115                 120                 125

Leu Gln Lys Gly Arg Ser Val Ser Ala Ala Asp Leu Ser Glu Ala Glu
    130                 135                 140

Pro Thr Leu Thr His Met Ser Ile Thr Arg Leu His Glu Gln Lys Leu

145                 150                 155                 160

Val Gln His Val Val Ser Gln Asn Cys Asp Gly Leu His Leu Arg Ser
                165                 170                 175

Gly Leu Pro Arg Thr Ala Ile Ser Glu Leu His Gly Asn Met Tyr Ile
            180                 185                 190

Glu Val Cys Thr Ser Cys Val Pro Asn Arg Glu Tyr Val Arg Val Phe
        195                 200                 205

Asp Val Thr Glu Arg Thr Ala Leu His Arg His Gln Thr Gly Arg Thr
    210                 215                 220

Cys His Lys Cys Gly Thr Gln Leu Arg Asp Thr Ile Val His Phe Gly
225                 230                 235                 240

Glu Arg Gly Thr Leu Gly Gln Pro Leu Asn Trp Glu Ala Ala Thr Glu
                245                 250                 255

Ala Ala Ser Arg Ala Asp Thr Ile Leu Cys Leu Gly Ser Ser Leu Lys
            260                 265                 270

Val Leu Lys Lys Tyr Pro Arg Leu Trp Cys Met Thr Lys Pro Pro Ser
        275                 280                 285

Arg Arg Pro Lys Leu Tyr Ile Val Asn Leu Gln Trp Thr Pro Lys Asp
    290                 295                 300

Asp Trp Ala Ala Leu Lys Leu His Gly Lys Cys Asp Asp Val Met Arg
305                 310                 315                 320

Leu Leu Met Ala Glu Leu Gly Leu Glu Ile Pro Ala Tyr Ser Arg Trp
                325                 330                 335

Gln Asp Pro Ile Phe Ser Leu Ala Thr Pro Leu Arg Ala Gly Glu Glu
            340                 345                 350

Gly Ser His Ser Arg Lys Ser Leu Cys Arg Ser Arg Glu Glu Ala Pro
        355                 360                 365

Pro Gly Asp Arg Gly Ala Pro Leu Ser Ser Ala Pro Ile Leu Gly Gly
    370                 375                 380

Trp Phe Gly Arg Gly Cys Thr Lys Arg Thr Lys Arg Lys Lys Val Thr
385                 390                 395                 400

<210>  9
<211>  17
<212>  DNA
<213>  artificial sequence

<220>
<223>  primer for ACC1

<400>  9
caggccggcc aggtttg                                                    17


<210>  10
<211>  20
<212>  DNA
<213>  artificial sequence

<220>
<223>  primer for ACC1

<400>  10
tccatgtgcc gagggttgat                                                 20


<210>  11
<211>  24
<212>  DNA
<213>  artificial sequence

<220>
<223>  primer for ACLY

<400>  11
gaggtggccc caactatcaa gagg                                            24


<210>  12
<211>  24
<212>  DNA
<213>  artificial sequence

<220>
<223>  primer for ACLY

<400>  12
cccgctggca ttaaggagga agtt                                            24


<210>  13
<211>  24
<212>  DNA
<213>  artificial sequence

<220>
<223>  primer for PGC-1a

<400>  13
tacaatgaat gcagcggtct tagc                                            24


<210>  14
<211>  22
<212>  DNA
<213>  artificial sequence

<220>
<223>  primer for PGC-1a

<400>  14
gaggagggtc atcgtttgtg gt                                              22


<210>  15

<211>  24
<212>  DNA
<213>  artificial sequence

<220>
<223>  primer for PGC-1b

<400>  15
   gccctggaaa gcccctgtga gagt                                          24


<210>  16
<211>  24
<212>  DNA
<213>  artificial sequence

<220>
<223>  primer for PGC-1b

<400>  16
gtgtggtggg tggcgtgagt cctg                                           24


<210>  17
<211>  19
<212>  DNA
<213>  artificial sequence

<220>
<223>  primer for sirt7

<400>  17
ccccggaccg ccatctcag                                                 19


<210>  18
<211>  23
<212>  DNA
<213>  artificial sequence

<220>
<223>  primer for sirt7

<400>  18
atctccaggc ccagttcatt cat                                            23